# EUROPEAN PATENT APPLICATION

(11) **EP 1 457 207 A1**
(43) Date of publication of application: **15.09.2004**
(21) Application number: 02790798.9
(22) Date of filing: 18.12.2002
(51) Int. Cl.: A61K 31/403, A61K 9/36, A61K 47/22, A61P 25/00, A61P 25/16, A61P 25/18, A61P 25/28

(54) **SOLID COMPOSITIONS CONTAINNG COMPOUNDS UNSTABLE TO OXYGEN AND METHOD FOR STABILIZATION THEREOF**

(30) Priority: 19.12.2001 JP 2001386309
(71) Applicant: Takeda Chemical Industries, Ltd., Osaka-shi, Osaka 541-0045 (JP)
(72) Inventor: YAMASHITA, Akio, Toyonaka-shi, Osaka 560-0003 (JP); HIRASHIMA, Naoki, Kamakura-shi, Kanagawa 247-0063 (JP); NONOMURA, Muneo, Toyonaka-shi, Osaka 560-0052 (JP)
(74) Representative: Rickard, Timothy Mark Adrian
(86) International application number: PCT/JP2002/013218
(87) International publication number: WO 2003/051355

(57) **Abstract**

The present invention aims at stabilizing a solid composition containing a nitrogenous fused-heterocycle compound unstable to oxygen to provide a stable pharmaceutical preparation. The stabilization can be attained by keeping the equilibrium moisture content at 10% or above and/or adding ascorbic acid or a salt thereof, by preliminarily applying a film coating free from light blocking agents, or by applying one or more packaging selected from among oxygen-barrier packaging, inert gas replacement packaging, vacuum packaging and sealing packaging with an oxygen absorber.

## Description

### Technical Field

The present invention relates to a solid composition containing a compound unstable to oxygen and a method for stabilizing the same.

### Background Art

Fused nitrogen-containing heterocyclic compounds have been used for various pharmaceutical products. Fused nitrogen-containing heterocyclic compounds wherein a benzene ring and a 4- to 7-membered saturated nitrogen-containing heterocyclic ring are fused, particularly isoindoline compounds having a benzofuran ring as a substituent group on the nitrogen atom, recently, have been investigated for use as an agent for promoting nerve regeneration and/or an agent for promoting differentiation of neural stem cells. Such compounds have drawn attention as potential therapeutic drugs for Alzheimer's disease or the like. As examples of such fused nitrogen-containing heterocyclic compounds having a nerve regeneration-promoting activity, the compounds described in WO 00/34262 are known. However, fused nitrogen-containing heterocyclic compounds, particularly the compounds wherein a benzene ring and a 7 or less membered nitrogen-containing ring are fused including isoindoline compounds, have larger distortion and are more unstable as compared with the compounds wherein a benzene ring and 8 or more membered nitrogen-containing ring are fused. For example, by a pulverization step or the like in drug manufacture process and drug formulation process, the surface area of a drug increases and thereby the area that may contact with oxygen increases. As a result, a phenomenon wherein the saturated rings of compounds are oxidized to release hydrogen and then changed to aromatic rings, or the like is caused. In' such a manner, these compounds in a solid state are unstable to oxygen and also unstable to light.

On the other hand, in general, compounds in pharmaceutical preparations (e.g. tablets, powders, fine granules, granules, capsules) have reduced stability as compared with the compounds alone, due to strong interaction with other components in the pharmaceutical formulation. Thus, at the time of production and with the lapse of time, the content of a compound in a pharmaceutical preparation usually decreases and the color of the pharmaceutical preparation usually changes remarkably. In order to solve such a problem of instability, in investigations of formulation, compatibility tests or the like are performed to select excipients having better compatibility and then by using the selected excipients, appropriate stabilization of pharmaceutical preparations may be attained. However, although such a technique is conventional, such formulation and stabilization strategy comprising combination with suitable excipients vary depending on the characteristic physical properties of compounds to be used. Therefore, it is necessary to examine individually on individual compounds and to select individually suitable excipients to individual compounds, so that even a person skilled in the art cannot easily obtain the suitable formulation and stabilization strategy.

### Objective of the Invention

The objective of the present invention is to stabilize a solid composition containing a compound unstable to oxygen. Particularly, the objective of the present invention is to stabilize a solid composition containing a fused nitrogen-containing heterocyclic compound unstable to oxygen and to obtain a stable pharmaceutical preparation.

### Summary of the Invention

In view of the above-mentioned situation, the present inventors investigated how to stabilize a fused nitrogen-containing heterocyclic compound unstable to oxygen, and as a result, have found that bulk of a fused nitrogen-containing heterocyclic compound could be stabilized by formulation into pharmaceutical preparations. The present inventors have also found that stabilization of a pharmaceutical composition containing a fused nitrogen-containing heterocyclic compound can be achieved by applying a coating for protection from light to the composition and controlling the equilibrium moisture content of the composition and, if necessary, further by (1) incorporating ascorbic acid or a salt thereof in the composition and/or (2) precoating the composition with a film that does not contain a light blocking agent, as so-called an anchor coating. Furthermore, the present inventors have found such stabilization method is also effective in combination with stabilization by packing. Based on these findings and further investigations, the present inventors have completed the present invention.

That is, the present invention provides:
(1) a solid composition containing a fused nitrogen-containing heterocyclic compound unstable to oxygen, which is stabilized by
   [1] maintaining an equilibrium moisture content of 10% or above in the solid composition and/or
   [2] incorporating ascorbic acid or a salt thereof in the solid composition;
(2) the solid composition described in the above (1), wherein the fused nitrogen-containing heterocyclic compound is a compound represented by the formula: 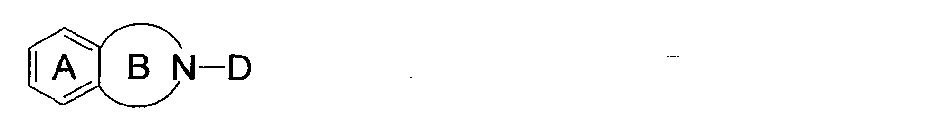 wherein, ring A is an optionally substituted benzene ring; ring B is a 4 to 7-membered nitrogen-containing heterocyclic ring which may be optionally substituted with halogen, an optionally substituted heterocyclic ring or an optionally substituted hydrocarbon group in addition to D; and D is a hydrogen atom, a heterocyclic group which may be optionally substituted and may optionally have a fused ring, or an optionally substituted hydrocarbon group, or a salt thereof (hereinafter, referred to as compound (I) in some cases);
(3) the solid composition described in the above (1), wherein the fused nitrogen-containing heterocyclic compound is an isoindoline compound;
(4) the solid composition described in the above (3), wherein the fused nitrogen-containing heterocyclic compound is (R)-(+)-5,6-dimethoxy-2-[2,2,4,6,7-pentamethyl-3-(4-methylphenyl)-2,3-dihydro-1-benzofuran-5-yl]isoindoline, (R)-(+)-5,6-dimethoxy-2-[2,2,4,6,7-pentamethyl-3-(1-methylethylphenyl)-2,3-dihydro-1-benzofuran-5-yl]isoindoline, (R)-(+)-5,6-dimethoxy-2-[2,2,4,6,7-pentamethyl-3-(4-bromophenyl)-2,3-dihydro-1-benzofuran-5-yl]isoindoline, or a salt thereof;
(5) the solid composition described in the above (2), which is coated with a film for protection from light;
(6) the solid composition described in the above (5), which is precoated with a film that does not contain a light blocking agent;
(7) a packed product obtained by packing the solid composition described in any one of the above (1) to (6) in one or more packaging forms selected from oxygen permeation-suppressing package, gas-replacement package, vacuum package and sealing package with an oxygen scavenger;
(8) a packed product obtained by packing a solid composition in nitrogen-replacement package, wherein the solid composition comprises a compound represented by the formula: 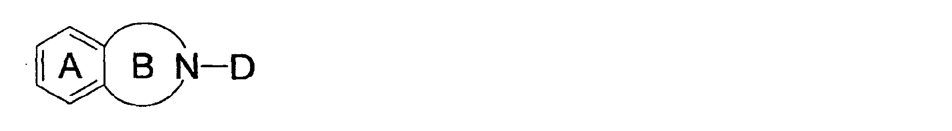 wherein, ring A is an optionally substituted benzene ring; ring B is a 4 to 7-membered nitrogen-containing heterocyclic ring which may be optionally substituted with halogen, an optionally substituted heterocyclic ring or an optionally substituted hydrocarbon group in addition to D; and D is a hydrogen atom, a heterocyclic group which may be optionally substituted and may optionally have a fused ring, or an optionally substituted hydrocarbon group, or a salt thereof, and ascorbic acid or a salt thereof; is coated with a film for protection from light without being precoated with a film; and has an equilibrium moisture content of 10% or above;
(9) a method for stabilizing a solid composition containing a fused nitrogen-containing heterocyclic compound unstable to oxygen, which comprises
   [1] maintaining an equilibrium moisture content of 10% or above in the solid composition,
   [2] incorporating ascorbic acid or a salt thereof in the solid composition, and/or
   [3] packing the solid composition in one or more packaging forms selected from oxygen permeation-suppressing package, gas-replacement package, vacuum package and sealing package with an oxygen scavenger;
(10) the method described in the above (9), wherein the fused nitrogen-containing heterocyclic compound is a compound represented by the formula: 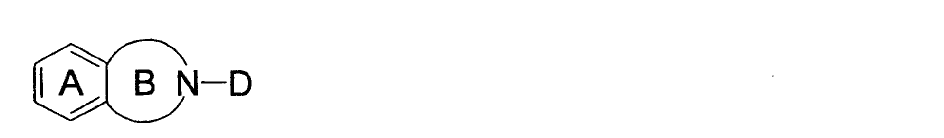 wherein, ring A is an optionally substituted benzene ring; ring B is a 4 to 7-membered nitrogen-containing heterocyclic ring which may be optionally substituted with halogen, an optionally substituted heterocyclic ring or an optionally substituted hydrocarbon group in addition to D; and D is a hydrogen atom, a heterocyclic group which may be optionally substituted and may optionally have a fused ring, or an optionally substituted hydrocarbon group, or a salt thereof;
(11) the method described in the above (9), wherein the fused nitrogen-containing heterocyclic compound is an isoindoline compound;
(12) the stabilization method described in the above (9), wherein the fused nitrogen-containing heterocyclic compound is (R)-(+)-5,6-dimethoxy-2-[2,2,4,6,7-pentamethyl-3-(4-methylphenyl)-2,3-dihydro-1-benzofuran-5-yl]isoindoline, (R)-(+)-5,6-dimethoxy-2-[2,2,4,6,7-pentamethyl-3-(3-methylethylphenyl)-2,3-dihydro-1-benzofuran-5-yl]isoindoline, (R)-(+)-5,6-dimethoxy-2-[2,2,4,6,7-pentamethyl-3-(4-bromophenyl)-2,3-dihydro-1-benzofuran-5-yl]isoindoline or a salt thereof;
(13) a stabilized solid composition which comprises [1] a compound unstable to oxygen and [2] an antioxidant that is less oxidizable than said compound and wherein an equilibrium moisture content of 10% or above is maintained;
(14) a packed product obtained by packing the composition described in the above (13) in one or more packaging forms selected from oxygen permeation-suppressing package, gas-replacement package, vacuum package, and sealing package with an oxygen scavenger; and
(15) a method for stabilizing a solid composition containing a compound unstable to oxygen, which comprises
   [1] maintaining an equilibrium moisture content of 10% or above in the solid composition,
   [2] incorporating an antioxidant that is less oxidizable than the compound in the solid composition, and/or
   [3] packing the solid composition in one or more packaging forms selected from oxygen permeation-suppressing package, gas-replacement package, vacuum package, and sealing package with an oxygen scavenger.

The present invention further provides:
(16) the pharmaceutical solid composition described in the above (2), wherein ring B is a 4- to 5-membered nitrogen-containing heterocyclic ring:
(17) a pharmaceutical solid composition as described in the above (1), wherein the fused nitrogen-containing heterocyclic compound is a compound represented by the formula; 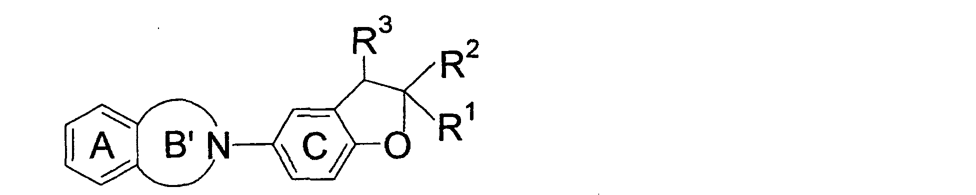 wherein, ring A is an optionally substituted benzene ring; R¹ and R² are independently a hydrogen atom or an optionally substituted hydrocarbon group; R³ is an optionally substituted aromatic group; ring B' is a 4 to 7-membered nitrogen-containing heterocyclic ring which may be optionally substituted with halogen or an optionally substituted hydrocarbon group; and ring C is an optionally further substituted benzene ring, or a salt thereof (hereinafter, referred to as compound (II) in some cases);
(18) the method as described in the above (9), wherein ring B is a 4- or 5-membered nitrogen-containing heterocyclic ring; and
(19) a method as described in the above (9), wherein the fused nitrogen-containing heterocyclic compound is the compound (II).

### Detailed Explanation of Invention

A compound unstable to oxygen, as used herein, includes a fused nitrogen-containing heterocyclic compound unstable to oxygen. As such a compound, the above-mentioned compound (I) and especially the compound (II) are exemplified. These compounds are also unstable to light. The compound (II) has an activity for promoting nerve regeneration and/or an activity for promoting differentiation of neural stem cells.

The ring A in the compound (I) is "an optionally substituted benzene ring". Examples of a "substituent" for the ring A include (1) a halogen atom (e.g. fluorine, chlorine, bromine, and iodine, (2) C₁₋₃ alkylenedioxy (e.g. methylenedioxy and ethylenedioxy), (3) nitro, (4) cyano, (5) optionally halogenated C₁₋₆ alkyl, (6) optionally halogenated C₂₋₆ alkenyl, (7) optionally halogenated C₂₋₆ alkynyl, (8) optionally halogenated C₃₋₆ cycloalkyl, (9) C₆₋₁₄ aryl (e.g. phenyl, 1-naphthyl, 2-naphthyl, biphenyl, 2-anthryl), (10) optionally halogenated C₁₋₆ alkoxy, (11) optionally halogenated C₁₋₆ alkylthio or mercapto, (12) hydroxy, (13) amino, (14) mono-C₁₋₆ alkylamino (e.g. methylamino and ethylamino), (15) mono-C₆₋₁₄ arylamino (e.g. phenylamino, 1-naphthylamino, and 2-naphthylamino), (16) di-C₁₋₆ alkylamino (e.g. dimethylamino and diethylamino), (17) di-C₆₋₁₄ arylamino (e.g. diphenylamino), (18) acyl, (19) acylamino, (20) acyloxy, (21) optionally substituted 5 to 7-membered saturated cyclic amino, (22) a 5 to 10-membered aromatic heterocyclic group (e.g. 2- or 3-thienyl, 2-, 3-, or 4-pyridyl, 2-, 3-, 4-, 5-, or 8-quinolyl, 1-, 3-, 4-, or 5-isoquinolyl, 1-, 2-, or 3-indolyl, 2-benzothiazolyl, 2-benzo[b]thienyl and benzo[b]furanyl), (23) sulfo, and (24) C₆₋₁₄ aryloxy (e.g. phenyloxy and naphthyloxy). The ring A may have 1 to 4 (preferably 1 or 2) substituents selected from the above substituents at the substitutable positions. If the ring A has 2 or more substituents, the substituents may be the same as of different from one another.

The above-mentioned "optionally halogenated C₁₋₆ alkyl" may be C₁₋₆ alkyl (e.g. methyl, ethyl, propyl, isopropyl, butyl, isobutyl, sec-butyl, tert-butyl, pentyl, and hexyl) which may optionally have 1 to 5, preferably to 3 halogen atoms (e.g. fluorine, chlorine, bromine, and iodine). Specific examples thereof include methyl, chloromethyl, difluoromethyl, trichloromethyl, trifluoromethyl, ethyl, 2-bromoethyl, 2,2,2-trifluoroethyl, pentafluoroethyl, propyl, 3,3,3-trifluoropropyl, isopropyl, butyl, 4,4,4-trifluorobutyl, isobutyl, sec-butyl, tert-butyl, pentyl, isopentyl, neopentyl, 5,5,5-trifluoropentyl, hexyl, 6,6,6-trifluorohexyl, and the like.

The above-mentioned "optionally halogenated C₂₋₆ alkenyl" may be C₂₋₆ alkenyl (e.g. vinyl, allyl, isopropenyl, butenyl, isobutenyl, and sec-butenyl) which may optionally have 1 to 5, preferably 1 to 3 halogen atoms (e.g. fluorine, chlorine, bromine, and iodine). Specific examples thereof include vinyl, allyl, isopropenyl, butenyl, isobutenyl, sec-butenyl, 3,3,3-trifluoro-1-propenyl, 4,4,4-trifluoro-1-butenyl, and the like.

The above-mentioned "optionally halogenated C₂₋₆ alkynyl" may be C₂₋₆ alkynyl (e.g. ethynyl, propargyl, butynyl, and 1-hexynyl) which may optionally have 1 to 5, preferably 1 to 3 halogen atoms (e.g. fluorine, chlorine, bromine, and iodine). Specific examples thereof include ethynyl, propargyl, butynyl, 1-hexynyl, 3,3,3-trifluoro-1-propynyl, 4,4,4-trifluoro-1-butynyl and the like.

The above-mentioned "optionally halogenated C₃₋₆ cycloalkyl" may be C₃₋₆ cycloalkyl (e.g. cyclopropyl, cyclobutyl, cyclopentyl, and cyclohexyl) which may optionally have 1 to 5, preferably 1 to 3 halogen atoms (e.g. fluorine, chlorine, bromine, and iodine). Specific examples thereof include cyclopropyl, cyclobutyl, cyclopentyl, cyclohexyl, 4,4-dichlorocyclohexyl, 2,2,3,3-tetrafluorocyclopentyl, 4-chlorocyclohexyl and the like.

The above-mentioned "optionally halogenated C₁₋₆ alkoxyl" may be C₁₋₆ alkoxy (e.g. methoxy, ethoxy, propoxy, isopropoxy, butoxy, isobutoxy, sec-butoxy, pentyloxy, and hexyloxy) which may optionally have 1 to 5, preferably 1 to 3 halogen atoms (e.g. fluorine, chlorine, bromine, and iodine). Specific examples thereof include methoxy, difluoromethoxy, trifluoromethoxy, ethoxy, 2,2,2-trifluoroethoxy, propoxy, isopropoxy, butoxy, 4,4,4-trifluorobutoxy, isobutoxy, sec-butoxy, pentyloxy, hexyloxy and the like.

The above-mentioned "optionally halogenated C₁₋₆ alkylthio" may be C₁₋₆ alkylthio (e.g. methylthio, ethylthio, propoylthio, isopropylthio, butylthio, sec-butylthio, and tert-butylthio) which may optionally, have 1 to 5, preferably 1 to 3 halogen atoms (e.g. fluorine, chlorine, bromine, and iodine). Specific examples thereof include methylthio, difluoromethylthio, trifluoromethylthio, ethylthio, propoylthio, isopropylthio, butylthio, 4,4,4-trifluorobutylthio, pentylthio, hexylthio, and the like.

The above-mentioned "acyl" includes formyl, carboxy, carbamoyl, C₁₋₆ alkyl-carbonyl (e.g. acetyl and propionyl), C₃₋₆ cycloalkyl-carbonyl (e.g. cyclopropylcarbonyl, cyclopentylcarbonyl, and cyclohexylcarbonyl), C₁₋₆ alkoxycarbonyl (e.g. methoxycarbonyl, ethoxycarbonyl, propoxycarbonyl, and tert-butoxycarbonyl), C₆₋₁₄ arylcarbonyl (e.g. benzoyl, 1-naphthoyl, and 2-naphthoyl), C₇₋₁₆ aralkyl-carbonyl (e.g. phenylacetyl and phenylpropionyl), C₆₋₁₄ aryloxy-carbonyl (e.g. phenoxycarbonyl), C₇₋₁₆ aralkyloxy-carbonyl (e.g. benzyloxycarbonyl and phenethyloxycarbonyl), 5 or 6-membered heterocyclic carbonyl (e.g. nicotinoyl, isonicotinoyl, 2-thenoyl, 3-thenoyl, 2-furoyl, 3-furoyl, morpholionocarbonyl, thiomorpholinocarbonyl, piperidinocarbonyl, and 1-pyrrolidinylcarbonyl), mono-C₁₋₆ alkyl-carbamoyl (e.g. methylcarbamoyl and ethylcarbamoyl), di-C₁₋₆ alkyl-carbamoyl (e.g. dimethylcarbamoyl, diethylcarbamoyl, and ethylmethylcarbamoyl), C₆₋₁₄ arylcarbamoyl (e.g. phenylcarbamoyl, 1-naphthylcarbamoyl, and 2-naphthylcarbamoyl), thiocarbamoyl, 5 or 6-membered hexacyclic carbamoyl (e.g. 2-pyridylcarbamoyl, 3-pyridylcarbamoyl, 4-pyridylcarbamoyl, 2-thienylcarbamoyl, and 3-thienylcarbamoyl), C₁₋₆ alkylsulfonyl (e.g. methylsulfonyl and ethylsulfonyl), C₆₋₁₄ arylsulfonyl (e.g. phenylsulfonyl, 1-naphthylsulfonyl, and 2-naphthylsulfonyl), C₁₋₆ alkylsulfinyl (e.g. methylsulfinyl and ethylsulfinyl), C₆₋₁₄ arylsulfinyl (e.g. phenylsulfinyl, 1-naphthylsulfinyl, and 2-naphthylsulfinyl), and the like.

The above-mentioned "acylamino" includes formylamino, C₁₋₆ alkyl-carbonylamino (e.g. acetylamino), C₆₋₁₄ arylcarbonylamino (e.g. phenylcarbonylamino and naphthylcarbonylamino), C₁₋₆ alkoxyl-carbonylamino (e.g. methoxycarbonylamino, ethoxycarbonylamino, propoxycarbonylamino, and butoxycarboylamino), C₁₋₆ alkylsulfonylamino (e.g. methylsulfonylamino, and ethylsulfonylamino), C₆₋₁₄ arylsulfonylamino (e.g. phenylsulfonylamino, 2-naphthylsulfonylamino, and 1-naphthylsulfonylamino), and the like.

The above-mentioned "acyloxy" includes C₁₋₆ alkylcarbonyloxy (e.g. acetoxy and propionyloxy), C₆₋₁₄ arylcarbonyloxy (e.g. benzoyloxy and naphthylcarbonyloxy), C₁₋₆ alkoxy-carbonyloxy (e.g. methoxycarbonyloxy, ethoxycarbonyloxy, propoxycarbonyloxy and butoxycarbonyloxy), mono-C₁₋₆ alkyl-carbamoyloxy (e.g. methylcarbamoyloxy and ethylcarbamoyloxy), di-C₁₋₆ alkyl-carbamoyloxy (e.g. dimethylcarbamoyloxy and diethylcarbamoyloxy), C₆₋₁₄ aryl-carbamoyloxy (e.g. phenylcarbamoyloxy, naphthylcarbamoyloxy), nicotinoyloxy, and the like.

The "5 to 7-membered saturated cyclic amino" for the above-mentioned "optionally substituted 5 to 7-membered saturated cyclic amino" includes morpholino, thiomorpholino, piperazin-1-yl, piperidino, pyrrolidin-1-yl, and the like. A "substituent" for the above-mentioned "optionally substituted 5 to 7-membered saturated cyclic amino" includes C₁₋₆ alkyl (e.g. methyl, ethyl, propyl, isopropyl, butyl, isobutyl, see-butyl, tert-butyl, pentyl, and hexyl), C₆₋₁₄ aryl (e.g. phenyl, 1-naphthyl, 2-naphthyl, biphenylyl, and 2-anthryl), a 5 to 10-membered aromatic heterocyclic group (e.g. 2- or 3-thienyl, 2-, 3-, or 4-pyridyl, 2-, 3-, 4-, 5-, or 8-quinolyl, 1-, 3-, 4-, or 5-isoquinolyl, 1-, 2-, or 3-indolyl, 2-benzothiazolyl, 2-benzo[b]thienyl, and benzo[b]furanyl), and the like. The above-mentioned "optionally substituted 5 to 7-membered saturated cyclic amino" may have 1 to 3 of these substituents.

The ring B in the compound (I) is a "4- to 7-membered nitrogen-containing heterocyclic ring" and includes azetidine, azetidinone, pyrrole (e.g. 1H-pyrrole), dihydropyrrole (e.g. 2,5-dihydro-1H-pyrrole), dihydropyridine (e.g. 1,2-dihydropyridine), tetrahydropyridine (e.g. 1,2,3,4-tetrahydropyridine), azepine (e.g. 1H-azepine), dihydroazepine (e.g. 2,3-dihydro-1H-azepine, 2,5-dihydro-1H-azepine, 2,7-dihydro-1H-azepine), tetrahydroazepine (e.g. 2,3,6,7-tetrahydro-1H-azepine, 2,3,4,7-tetrahydro-1H-azepine), and the like.

The ring B may be optionally substituted with "halogen", an, "optionally substituted heterocyclic ring" or an "optionally substituted hydrocarbon group" in addition to D.

The "halogen" includes fluorine, chlorine, bromine, and iodine.

A "heterocyclic group" for the "optionally substituted heterocyclic ring" includes 5- to 14-membered heterocyclic groups (aromatic heterocyclic groups, saturated or unsaturated non-aromatic heterocyclic groups) containing 1 to 4 heteroatoms selected from a nitrogen atom, a sulfur atom and an oxygen atom in addition to carbon atoms.

The "aromatic heterocyclic group" includes 5- to 14-membered, preferably 5- to 10-membered aromatic heterocyclic groups containing one or more (e.g. 1 to 4) heteroatoms selected from a nitrogen atom, a sulfur atom and an oxygen atom in addition to carbon atoms and specifically, monovalent groups obtained by eliminating any optional hydrogen atom from aromatic heterocyclic rings such as thiophene, benzothiophene, benzofuran, benzimidazole, benzoxazole, benzothiazole, benzisothiazole, naphtho[2,3-b]thiophene, furan, isoindolizine, xanthrene, phenoxathiine, pyrrole, imidazole, pyrazole, pyridine, pyrazine, pyrimidine, pyridazine, indole, isoindole, 1H-indazole, purine, 4H-quinolizine, isoquinoline, quinoline, phthalazine, naphthyridine, quinoxaline, quinazoline, cinnoline, carbazole, β-carboline, phthanthridine, acridine, phenazine, thiazole, isothiazole, phenothiazine, oxazole, isoxazole, furazan, and phenoxazine, or rings formed by condensing these rings (preferably monocyclic rings) with one or more (preferably 1 or 2) aromatic rings (e.g. benzene ring).

Preferable examples of the "aromatic heterocyclic group" include 5- or 6-membered aromatic heterocyclic groups which may be fused with one benzene ring and specifically, 2-, 3- or 4-pyridyl, 2-, 3-, 4-, 5- or 8-quinolyl, 1-, 3-, 4- or 5-isoquinolyl, 1-, 2- or 3-indolyl, 2-benzothiazolyl, 2-benzo[b]thienyl, benzo[b]furanyl, and 2- or 3-thienyl. More preferable examples thereof are 2-or 3-thienyl, 2-, 3- or 4-pyridyl, 2- or 3-quinolyl, 1-isoquinolyl, 1- or 2-indolyl, 2-benzothiazolyl, and the like.

The "non-aromatic heterocyclic ring" includes 3 to 8-membered (preferably 5 or 6-membered) saturated or unsaturated (preferably saturated) non-aromatic heterocyclic groups (aliphatic heterocyclic groups) such as oxiranyl, azetidinyl, oxetanyl, thietanyl, pyrrolidinyl, tetrahydrofuryl, thiolanyl, piperidyl, tetrahydropyranyl, morpholinyl, thiomorpholinyl and piperazinyl.

A "substituent" for the "optionally substituted heterocyclic group" includes those similar to the "substituent" for the above-mentioned ring A. The "optionally substituted heterocyclic group" may have 1 to 5, preferably 1 to 3 the substituents at the substituable positions and if it has 2 or more substituents, the substituents may be the same as of different from one another.

A "hydrocarbon group" for the "optionally substituted hydrocarbon group" includes chain or cyclic hydrocarbon groups (e.g. alkyl, alkenyl, alkynyl, cycloalkyl, and aryl). Among them, chain or cyclic hydrocarbon groups containing 1 to 16 carbon atoms are preferred.

The "alkyl" is preferably C₁₋₆ alkyl (e.g. methyl, ethyl, propyl, isopropyl, butyl, isobutyl, sec-butyl, tert-butyl, pentyl, and hexyl).

The "alkenyl" is preferably C₂₋₆ alkenyl (e.g. vinyl, allyl, isopropenyl, butenyl, isobutenyl, sec-butenyl).

The "alkynyl" is preferably C₂₋₆ alkynyl (e.g. ethynyl, propargyl, butynyl, and 1-hexynyl).

The "cycloalkyl" is preferably C₃₋₆ cycloalkyl (e.g. cyclopropyl, cyclobutyl, cyclopentyl, and cyclohexyl).

The "aryl" is preferably C₆₋₁₄ aryl (e.g. phenyl, 1-naphthyl, 2-naphthyl, biphenylyl, and 2-anthryl).

A "substituent" for the "optionally substituted hydrocarbon group" includes those similar to the substituents for the above-mentioned ring A. For example, the "optionally substituted hydrocarbon group" may have 1 to 5, preferably 1 to 3 of the above-mentioned substituents at the substitutable positions and if it has 2 or more substituents, the substituents may be the same as or different from one another.

Specific examples of a group represented by the formula: 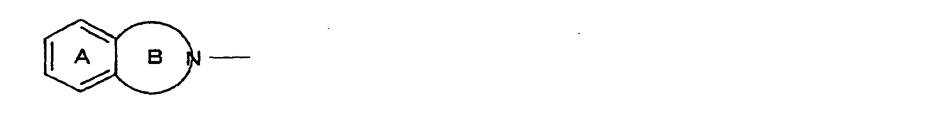 wherein each symbol is as defined above, include groups represented by the following formulas: 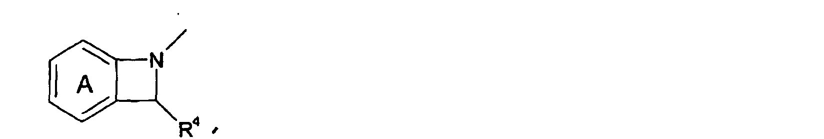 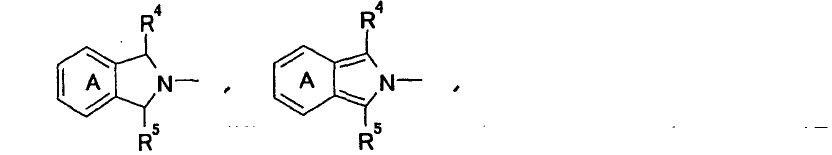 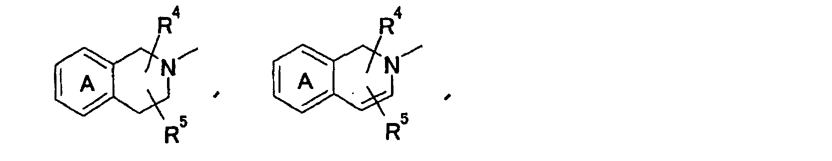 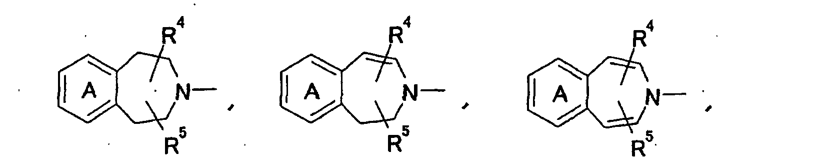 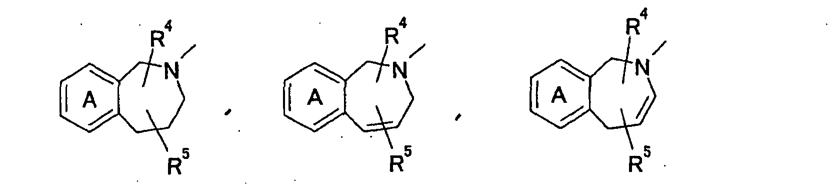 wherein, R⁴ and R⁵ may be the same or different and each is a hydrogen atom, halogen, or an optionally substituted hydrocarbon group, and ring A is as defined above; preferably groups represented by the following formulas: 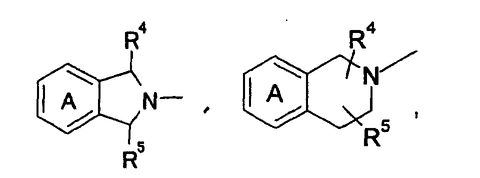 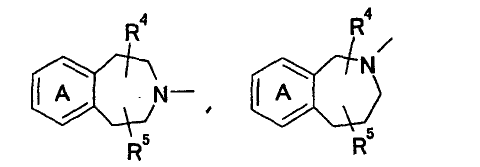 wherein each symbol is as defined above; more preferably groups represented by the following formulas: 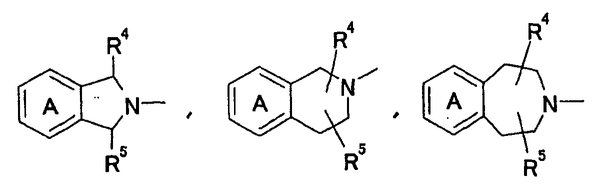 wherein each symbol is as defined above; and most preferably groups represented by the following formula: 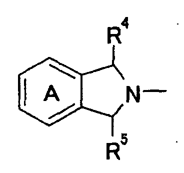 wherein each symbol is as defined above.

Representative compounds which may be used in the present invention include isoindoline compounds, that is, compounds having isoindoline as the partial structure.

The "halogen" or "optionally substituted hydrocarbon group" represented by R⁴ and R⁵ include those similar to the "halogen" or "optionally substituted hydrocarbon group" exemplified as the "substituent" for the above-mentioned ring B, respectively.

D in the compound (I) is a "hydrogen atom", a "heterocyclic group which may be optionally substituted and may optionally have a fused ring" or an "optionally substituted hydrocarbon group".

A "substituent" and "heterocyclic group" for the "heterocyclic group which may be optionally substituted and may optionally have a fused ring" include those similar to groups exemplified with respect to the above-mentioned ring B. The "fused ring" includes monovalent groups obtained by eliminating any optional hydrogen from rings formed by condensing aromatic heterocyclic rings with one or more (preferable 1 or 2) aromatic rings (e.g. a benzene ring). Specific examples thereof are 2-, 3-, 4-, 5- or 8-quinolyl, 1-, 3-, 4- or 5-isoquinolyl, 1-, 2- or 3-indolyl, 2-benzothiazolyl, and 2-benzo[b]thienyl, benzo[b]furanyl and the like.

The "optionally substituted hydrocarbon group" represented by D includes those similar to the "optionally substituted hydrocarbon group" which is a "substituent" for the above-mentioned ring B.

The ring A of the compound (II) includes those similar to the ring A of the compound (I).

The "optionally substituted hydrocarbon group" represented by R¹ and R² in the compound (II) includes those similar to the "optionally substituted hydrocarbon groups" for the ring B in the compound (I).

The "optionally substituted aromatic group" represented by R³ includes "optionally substituted C₆₋₁₄ aryl" and examples thereof are C₆₋₁₄ aryl group such as phenyl, 1-naphthyl, 2-naphthyl, biphenylyl and anthryl, and the like. A "substituent" for the "optionally substituted C₆₋₁₄ aryl" includes those similar to the above-mentioned "substituents" for the "optionally substituted hydrocarbon group" for ring B of the compound (I). The number of substituents which the "optionally substituted C₆₋₁₄ aryl" may have is also similar to that of the "optionally substituted hydrocarbon group" for ring B of the compound (I).

The ring B' of the compound (II) includes those similar to the ring B of the above-mentioned compound (I).

The ring C of the compound (II) is a benzene ring which may optionally have a substituent group in addition to ring B' and the "substituent" includes those similar to the above-mentioned substituents for the ring A of the compound (I).

Particularly, the present invention can be preferably applied to isoindoline compounds, for example, (R)-(+)-5,6-dimethoxy-2-[2,2,4,6,7-pentamethyl-3-(4-methylphenyl)-2,3-dihydro-1-benzofuran-5-yl]isoindoline, [(R)-5,6-dimethoxy-2-[2,2,4,6,7-pentamethyl-3-(4-methylphenyl)benzofuran-5-yl]-2,3-dihydro-1H-isoindole, (R)-(+)-5,6-dimethoxy-2-[2,2,4,6,7-pentamethyl-3-(1-methylethylphenyl)-2,3-dihydro-1-benzofuran-5-yl]isoindoline, (R)-(+)-5,6-dimethoxy-2-[2,2,4,6,7-pentamethyl-3-(4-bromophenyl)-2,3-dihydro-1-benzofuran-5-yl]isoindoline, and their salts.

Examples of salts of the above-mentioned compounds (I) and (II) may be metal salts, ammonium salts, or salts with organic bases in the case that the compounds have an acidic group such as -COOH or the like, and salts with inorganic acid, organic acid, or basic or acidic amino acid as well as intermolecular salts in the case that the compounds have a basic group such as -NH₂ or the like. Preferable examples of the metal salts include alkali metal salts such as a sodium salt and a potassium salt; alkaline earth metal salts such as a calcium salt, a magnesium salt and a barium salt; and an aluminum salt. Preferable examples of the salts with organic bases include salts with trimethylamine, triethylamine, pyridine, picoline, ethanolamine, diethanolamine, triethanolamine, dicyclohexylamine, and N,N-dibenzylethylenediamine. Preferable examples of the salts with inorganic acid include salts with hydrochloric acid, hydrobromic acid, nitric acid, sulfuric acid, and phosphoric acid. Preferable examples of the salts with organic acid include salts with formic acid, acetic acid, trifluoroacetic acid, fumaric acid, oxalic acid, tartaric acid, maleic acid, citric acid, succinic acid, malic acid, methanesulfonic acid, benzenesulfonic acid, and p-toluenesulfonic acid. Preferable examples of salts with basic amino acid include salts with arginine, lysine, and ornithine. Preferable examples of salts with acidic amino acid include salts with aspartic acid and glutamic acid.

Among them, pharmacologically acceptable salts are preferred and include, in the case that acidic functional groups exist in the compounds, inorganic salts such as alkali metal salts (e.g. a sodium salt and a potassium salt) and alkaline earth salts (e.g. a calcium salt, a magnesium salt, and a barium salt), and ammonium salts; and in the case basic functional groups exist in the compounds, inorganic salts such as hydrochloride, sulfate, phosphate and hydrobromide, and organic salts such as acetate, maleate, fumarate, succinate, methanesulfonate, p-toluenesulfonate, citrate and tartarate.

The compound (I) and the compound (II) can be produced by well-known methods, for example, methods described in WO 98/55454, WO 00/36262, WO 95/29907, JP-A 5-194466, USP 4,881,967, USP 4,212,865 and Tetrahedron Letters, vol. 37, no. 51, pp.9183-9186 (1996), or similar methods to these methods.

The solid composition, as used herein, includes pharmaceutical preparations (e.g. tablets, powders, fine granules, granules, capsules) containing the above-mentioned fused nitrogen-containing heterocyclic compound unstable to oxygen as an active component.

As the packed product, for example, the products obtained by packing the above-mentioned pharmaceutical preparations in prescribed packaging forms are exemplified.

Hereinafter, the solid composition, the packed product and the stabilization method of the present invention are explained.

One of stabilization methods of the present invention is accomplished by maintaining the equilibrium moisture content (ERH) of the solid composition at a given level. Since the component of a solid composition generally becomes more unstable with an increase in the water content of the solid composition, stabilization of a solid composition is usually carried out by lowering the water content. However, the inventors of the present invention unexpectedly found that oxidation of a fused nitrogen-containing heterocyclic compound unstable to oxygen can be suppressed by controlling the ERH of a solid composition so as to prevent a decrease in the ERH and as a result, the solid composition can be stabilized. A method for controlling the ERH of a solid composition is not particularly limited and may be any method as long as the method is capable of controlling the ERH of the final solid composition so as to be 10% or more, preferably 20% or more, and more preferably 30% or more as measured using, for example, Rotronic Hygrpskop DT (Rotronic Co.) under the conditions shown in the following Experimental Example 3. The control of ERH may be, for example, process control during production of a solid composition or control of a water content by an additional step such as a humidification step after the production. Alternatively, a solid composition is packed and humidified in a package to allow the ERH to reach to a given level. "Maintaining ERH" does not necessarily mean positive humidification. For example, if ERH is at the desired level or above, humidifying process is not required.

Another stabilization method of the present invention is accomplished by incorporating an antioxidant that is less oxidizable than the "compound unstable to an acid" in the solid composition and maintaining the ERH of the solid composition at a given level or above. The antioxidant used is usually a compound that is more easily oxidizable than a compound to be prevented from oxidation, in order to allow the antioxidant to consume oxygen in preference to the compound to be prevented from oxidation. However, surprisingly, the present inventors have found that oxidation of a "compound unstable to an acid" can be prevented by combining a compound that is more easily oxidizable than the compound to be prevented from oxidation and maintaining the ERH at a given level or above.

"Less oxidizable" means that the rate of a decrease in the weight of a substance is lower under the common laboratory environment (e.g. under atmospheric air, 25°C, and 50% humidity). The rate is expressed as a percentage of a decrease in the weight of a substance after leaving it under the common laboratory environment for a week.

The ERH level may be maintained at the above-mentioned level or above by a similar method to the above-mentioned method.

Such an antioxidant is not particularly limited as long as it is less oxidizable than a "compound unstable to oxygen" to be prevented from oxidation and may be any usually used antioxidant. Such an antioxidant includes ascorbic acid or a salt thereof (e.g. a sodium salt, a calcium salt, a magnesium salt, a potassium salt, a basic amino acid salt, a meglumine salt and the like), sodium nitrite, L-ascorbic acid stearic acid ester, sodium hydrogen sulfite, sodium sulfite, a salt of edetic acid (e.g. a sodium salt, a potassium salt, and a calcium salt), erithorbic acid, cysteine hydrochloride, citric acid, tocopherol acetate, cysteine, potassium dichloroisocyanurate, dibutylhydroxytoluene (BHT), soybean lecithin, sodium thioglycolate, thioglycerol, tocopherol (Vitamin E), d-δ-tocopherol, sodium formaldehyde sulfoxylate, ascorbic palmitate, sodium pyrosulfite, butylhydroxyanisole (BHA), 1,3-butylene glycol, benzotriazole, pentaerythrityl tetrakis[3-(3,5-di-tert-butyl-4-hydroxyphenyl)propionate], propyl gallate, and 2-mercaptobenzimidazole.

The antioxidant to be used in the present invention, as is clear from the above, may be selected depending on the "compound unstable to oxygen" to be prevented from oxidation.

If the "compound unstable to oxygen" is a fused nitrogen-containing heterocyclic compound, particularly the compound (I) or the compound (II), preferable examples of the antioxidant include ascorbic acid and a salt thereof (e.g. a sodium salt, a calcium salt, a magnesium salt, a potassium salt, a basic amino acid salt, and a meglumine salt), sodium nitrite, sodium hydrogen sulfite, sodium sulfite, a salt of edetic acid (e.g. a sodium salt, a potassium salt, and a calcium salt), erithorbic acid, cysteine hydrochloride, citric acid, cysteine, potassium dichloroisocyanurate, sodium thioglycolate, thioglycerol, sodium formaldehyde sulfoxylate, sodium pyrosulfite, and 1,3-butylene glycol, and the particularly preferable examples are ascorbic acid and a salt thereof (e.g. a sodium salt, a calcium salt, a magnesium salt, a potassium salt, a basic amino acid salt, and a meglumine salt).

These antioxidants may be used alone or two or more of them may be used in a combination.

These antioxidants may be mixed with other components of the solid composition in any proper step of formulation process by well-known methods. Although the amount used of the antioxidant is not particularly limited, it is usually 0.01% or above, preferably 0.1% or above, more preferably 1.0% or above, and most preferably 5.0% or above in the total weight of the solid composition. The antioxidant may be in any form as long as it is incorporated in the solid composition.

If the "compound unstable to oxygen" is a fused nitrogen-containing heterocyclic compound, particularly the compound (I) or the compound (II), it is preferable that the solid composition is coated for protection from light by well-known methods. A coating base of the coating for protection from light includes hydroxypropylmethyl cellulose, ethyl cellulose, hydroxymethyl cellulose, hydroxypropyl cellulose, polyoxyethylene glycol, Tween 80, Pluronic F68, cellulose acetate phthalate, hydroxypropylmethyl cellulose phthalate, hydroxymethyl cellulose acetate succinate, Eudragit (Rohm Pharma, West Germany, methacrylic acid-acrylic acid copolymer), cetanol, polyvinyl alcohol and zein. A light blocking agent of the coating for protection from light includes titanium dioxide and talc. Other ingredients of the coating agent include yellow ferric oxide, red ferric oxide, polyethylene glycol, riboflavin, carboxyvinyl polymer, hydroxyethyl cellulose, cellulose acetate, gelatin, maltitol and serac. Talc can work as a light blocking agent and can be also used as a plasticizer.

Further, in order to ensure more improved stability in the presence of oxygen and light, the solid composition may be precoated with a film that does not contain a light blocking agent, so-called an anchor coating, prior to the coating for protection from light. For such an anchor coating, those exemplified as the film base of the above-mentioned film coating can be used. For example, in the case of a tablet, the surface of a tablet is precoated with hydroxypropylmethyl cellulose or the like so as to attain a thickness of 0.1 to 30 mg/cm², preferably 1 to 20 mg/cm², and more preferably 3 to 10 mg/cm², and the precoated tablet is then coated with a film coating solution comprising hydroxypropylmethyl cellulose, Macrogol 6000, titanium dioxide, a pigment and the like so as to attain a thickness of about 3 to 10 mg/cm². The coating tablet thus obtained shows excellent stability with little change in the appearance and little decrease in the content of the active component even after being stored for a long time.

The purposes of the film coating may also include masking of taste, enteric property or durability.

Generally, a fused nitrogen-containing heterocyclic compound unstable to oxygen by itself is quickly oxidized in the presence of oxygen. The stability of a compound in a solid composition is usually lower than that of the compound by itself, as described above. However, unexpectedly, the present inventors found that oxidation of such a compound can be suppressed by formulation using a conventional method.

The solid composition of the present invention can be produced by a well-known formulation process (e.g. the methods described in General Rules for Preparations of the Japanese Pharmacopoeia 10th edition) and can be formulated into dosage forms suitable for oral administration such as tablets, capsules, powders, granules and fine granules. For example, in the case of a tablet, a compound unstable to oxygen is mixed with an excipient and a disintegrant and further mixed with a binder to form granules, and the granules are then mixed with a lubricant and compressed into tablets. In the case of a granule, the granule can be produced by extrusion granulation in a similar manner to the above-mentioned tablet, or fluidized bed granulation. The granule can be also produced by coating nonpareils (containing 75% (W/W) of white sugar and 25% (W/W) of cornstarch) with powder containing a compound unstable to oxygen and additives (e.g. white sugar, cornstarch, crystalline cellulose, hydroxypropyl cellulose, methyl cellulose, hydroxypropyl cellulose, and polyvinylpyrrolidone) while spraying water or a binder solution (concentration: about 0.5 to 70% (W/V)) of white sugar, hydroxypropyl cellulose, hydroxypropylmethyl cellulose or the like. In the case of a capsule, components are mixed simply and then filled in a capsule.

The solid composition of the present invention may contain a pharmacologically acceptable carrier or an additive in addition to the above-mentioned antioxidant. A pharmacologically acceptable carrier or an additive to be employed in production of the solid composition of the present invention includes various organic or inorganic carrier substances that are conventionally used as pharmaceutical material, for example, excipients, lubricants, binders and disintegrants for solid preparations; and solvents, solubilizing agents, suspending agents, isotonic agents, buffers and soothing agents for liquid preparations. If necessary, conventional additives such as preservatives, coloring agents, sweeteners, adsorbents, wetting agents and the like may be also used.

The excipients include lactose, white sugar, D-mannitol, starch, cornstarch, crystalline cellulose and light anhydrous silicic acid.

The lubricants include magnesium stearate, calcium stearate, talc and colloidal silica.

The binders include crystalline cellulose, white sugar, D-mannitol, dextrin, hydroxypropyl cellulose, hydroxypropyl methyl cellulose, polyvinylpyrrolidone, starch, sucrose, gelatin, methyl cellulose and sodium carboxymethyl cellulose.

The disintegrants include starch, carboxymethyl cellulose, calcium carboxymethyl cellulose, crosscarmelose sodium, carboxymethyl starch sodium and L-hydroxypropyl cellulose.

The solvents include water for injection, alcohol, propylene glycol, macrogol, sesame oil, corn oil and olive oil.

The solubilizing agents include polyethylene glycol, propylene glycol, D-mannitol, benzyl benzoate, ethanol, trisaminomethane, cholesterol, triethanolamine, sodium carbonate and sodium citrate.

The suspending agents include surfactants such as stearyltriethanolamine, sodium lauryl sulfate, laurylaminopropionic acid, lecithin, benzalkonium chloride, benzethonium chloride and glycerin monostearate; and hydrophilic polymers such as polyvinyl alcohol, polyvinylpyrrolidone, sodium carboxymethyl cellulose, methyl cellulose, hydroxymethyl cellulose, hydroxyethyl cellulose and hydroxypropyl cellulose.

The isotonic agents include glucose, D-sorbitol, sodium chloride, glycerin and D-mannitol.

The buffers include buffer solutions such as phosphate, acetate, carbonate and citrate buffer.

The soothing agents include benzyl alcohol.

The preservatives include p-hydroxybenzoic acid esters, chlorobutanol, benzyl alcohol, phenethyl alcohol, dehydroacetic acid and sorbic acid.

Another stabilization methods of the present invention is accomplished by taking a packaging form such as oxygen permeation-suppressing package, a method (gas replacement package) for replacing air with gas other than oxygen (e.g. nitrogen gas, argon gas, or carbon dioxide), vacuum package, oxygen scavenger-enclosing package, and the like. Such a packaging form leads to a decrease in the oxygen amount that may directly contact with the solid composition, and thereby the solid composition can be stabilized. In the case that an oxygen scavenger is enclosed, the solid composition may be packed in an oxygen-permeable package at first and then the packed product may be further packed in another package. To the allowable extent, the above-mentioned packaging forms can be combined with one another. For example, the oxygen permeation-suppressing package, gas replacement package and sealing package with an oxygen scavenger can be combined with one another.

Combination of the above-mentioned stabilization methods makes further stabilization possible.

Incidentally, in the case that the "compound unstable to oxygen" is the compound (I) or the compound (II) and nitrogen gas-replacement package is employed, stabilization can be achieved even without an anchor coating to the same extent as that in the case with an anchor coating.

Among the fused nitrogen-containing heterocyclic compounds used in the present invention, for example, the compound (II) is useful for mammalian (e.g. mice, rats, hamsters, rabbits, cats, dogs, bovines, sheeps, monkeys, humans, and the like) as a substance for promoting growth of stem cells (e.g. embryonic stem cells, neural stem cells and the like) or a substance for promoting differentiation of neural precursor cells; or as a neurotrophic factor-like substance, a neurotrophic factor activation-enhancing substance or a neurodegeneration-inhibiting substance, and it suppresses neural cell death and promotes regeneration of the nerve tissues or function by neurotization and neural axon extension. Further, the compound (II) is also useful in preparation of neural stem cells or neural cells (including neural precursor cells) from fetal brain or patient brain tissues and embryonic stem cells for transplantation treatment, as well as promotes engraftment, differentiation and functional expression of neural stem cells or neural cells after the transplantation.

Accordingly, stem cells and/or neural precursor cells proliferation- and/or differentiation-promoting agents comprising the compound (II) are effective against neurodegenerative diseases (e.g. Alzheimer's disease, Parkinson's disease, amyotrophic lateral sclerosis (ALS), Huntington's disease, spinocerebellar degeneration and the like), psychoneurosis diseases (e.g. schizophrenia), head trauma, spinal cord injury, cerebrovascular disorder, cerebrovascular dementia, and the like and is usable as an agent for preventing or treating such central nervous system disorders.

The compound (II) has low toxicity and can be administered orally and safely as it is or in the form of the above-mentioned solid composition obtained by mixing with a pharmacologically acceptable carrier by a known means.

The content of the compound (II) in the composition of the present invention is about 0.01 to 100% by weight of the total weight of the composition.

Although a dose of the composition of the present invention varies depending on a subject to be administered, a disease, and the like, it is about 0.1 to 20 mg /kg body weight, preferably about 0.2 to 10 mg/kg body weight, and more preferably about 0.5 to 10 mg/kg body weight of the compound (II) as an active component, when it is orally administered to an adult as a therapeutic agent for Alzheimer's disease. The dose may be administered once a day or more than once a day in several divided portions.

### Examples

The present invention is explained further in details with reference to Reference Examples, Examples and Experimental Examples, which are not intended to limit the present invention.

### Reference Example 1 (Compound A)

### (R)-(+)-5,6-dimethoxy-2-[2,2,4,6,7-pentamethyl-3-(4-methylphenyl)-2,3-dihydro-1-benzofuran-5-yl]isoindoline

Under argon atmosphere, 4,5-dimethoxyphthalic anhydride (4.43 g, 21,3 mmol) was added to a solution of (+)-2,2,4,6,7-pentamethyl-3-(4-methylphenyl)-2,3-dihydro-1-benzofuran-5-amine (6.00 g, 20.3 mmol) in tetrahydrofuran (50 mL) and the mixture was heated under reflux for 3 hours. The reaction mixture was cooled to room temperature and 1-ethyl-3-(3-dimethylaminopropyl)carbodiimide (WSC) hydrochloride (4.67 g, 24.4 mmol) and 1-hydroxy-1H-benzotriazole (HOBt) monohydrate (3.74 g, 24.4 mmol) were added. The resulting mixture was heated under reflux for 14 hours and then cooled to room temperature. To the reaction mixture were added water and an 8N aqueous solution of sodium hydroxide and the product was extracted twice with ethyl acetate. The extract was washed with an aqueous solution of saturated sodium hydrogen carbonate, dried over magnesium sulfate, filtered, and then concentrated under reduced pressure to obtain a crude product of (+)-5,6-dimethoxy-2-[2,2,4,6,7-pentamethyl-3-(4-methylphenyl)-2,3-dihydro-1-benzofuran-5-yl]-1H-isoindol-1,3(2H)-dione (8.40 g). To a solution of aluminum chloride (13.6 g, 102 mmol) in tetrahydrofuran (60 mL) was added Lithium aluminum hydride (3.87 g, 102 mmol) and stirred for 10 minutes. A solution of the above-mentioned crude product in tetrahydrofuran (30 mL) was added thereto and the mixture was heated under reflux for 3 hours. After cooled to room temperature, to the reaction mixture was added water and the mixture was then extracted twice with ethyl acetate. The extract was washed with a 1N aqueous solution of sodium hydroxide, dried over magnesium sulfate, filtered, and then concentrated under reduced pressure. The residue was subjected to silica gel column chromatography (hexane-ethyl acetate 8 : 1) to obtain the title compound (6.23 g, yield 68%). Melting point: 157 to 159°C. [α]_{D} = +62.3° (c = 0.488, methanol)
¹H-MMR(CDCl₃)δ:1.02 (3H,s), 1.51 (3H,s), 1.76 (3H,s), 2.17 (3H,s), 2.18 (3H,s), 2.31 (3H,s), 3.87 (6H,s), 4.10 (1H,s), 4.45 (4H,s), 6.70-7.15(6H,m).

### Experimental Example 1

After the bulk powder of the compound A and sodium ascorbate were left at 40°C and 75% RH in atmospheric air for 1 month, their residual ratios were measured. As a result, the residual ratio of the compound A was 89.7% (W/W) and the residual ratio of sodium ascorbate was 99.0% (W/W).

The quantitative determination of the compound A was carried out by a HPLC method under the following conditions:
solvent: acetonitrile,
measurement wavelength: 287 nm,
column: CHIRALCEL OJ-R 4.6 × 150 mm (manufactured by Daicel Chemical Industries, Ltd. CPI Co.),
mobile phase: a mixed (16 : 9) solution of acetonitrile/10 mM ammonium acetate aqueous solution,
oven temperature: around 25°C.

The quantitative determination of sodium ascorbate was carried out by iodine titration method (solvent: metaphosphoric acid solution (1 → 50), indicator: starch reagent solution).

### Example 1

The bulk powder of the compound A (1.8 g), D-mannitol (44.64 g), crosscarmelose sodium (2.7 g), and then a solution (4.32 g) of hydroxypropyl cellulose (1.62 g) were put in a mortar and then kneaded with a pestle. All the resulting wet kneaded mixture was dried in a vacuum drier (manufactured by Irie Seisakusho Co., Ltd.) to obtain a granule. The granule (45.12g) was pulverized in a mortar with a pestle and sieved through a No. 20 sieve to obtain a sized granule. The obtained sized granule (42.3 g) was mixed with crosscarmelose sodium (2.25 g) and magnesium stearate (0.45 g) in a polyethylene bag. The mixed powder thus obtained was compressed into a tablet with a universal testing machine (manufactured by Shimadzu Corp.) to obtain a plain tablet.

### Example 2

In a similar manner to Example 1, the bulk powder of the compound B (1.8 g), D-mannitol (44.64 g), crosscarmelose sodium (2.7 g), and then a solution (4.32 g) of hydroxypropyl cellulose (1.62 g) were put in a mortar and then kneaded with a pestle. All the resulting wet kneaded mixture was dried in a vacuum drier (manufactured by Irie Seisakusho Co., Ltd.) to obtain a granule. The granule (45.12g) was pulverized in a mortar with a pestle and sieved through a No. 20 sieve to obtain a sized granule. The obtained sized granule (42.3 g) was mixed with crosscarmelose sodium (2.25 g) and magnesium stearate (0.45 g) in a polyethylene bag. The mixed powder thus obtained was compressed into a tablet with a universal testing machine (manufactured by Shimadzu Corp.) to obtain a plain tablet.

### Example 3

In a similar manner to Example 1, the bulk powder of the compound C (1.8 g), D-mannitol (44.64 g), crosscarmelose sodium (2.7 g), and then a solution (4.32 g) of hydroxypropyl cellulose (1.62 g) were put in a mortar and then kneaded with a pestle. All the resulting wet kneaded mixture was dried in a vacuum drier (manufactured by Irie Seisakusho Co., Ltd.) to obtain a granule. The granule (45.12g) was pulverized in a mortar with a pestle and sieved through a No. 20 sieve to obtain a sized granule. The obtained sized granule (42.3 g) was mixed with crosscarmelose sodium (2.25 g) and magnesium stearate (0.45 g) in a polyethylene bag. The mixed powder thus obtained was compressed into a tablet with a universal testing machine (manufactured by Shimadzu Corp.) to obtain a plain tablet.

### Reference Example 2

In purified water (1,800 g) titanium dioxide (90 g), yellow ferric oxide (3.6 g) and red ferric oxide (3.6 g) were dispersed. In purified water (3,600 g) hydroxypropylmethyl cellulose 2910 (TC-5) (412.8 g) and Macrogol 6000 (90 g) were dissolved. The resulting dispersion and the resulting solution were mixed to obtain a coating agent.

### Reference Example 3

In purified water (5,400 g) hydroxypropylmethyl cellulose 2910 (TC-5) (600 g) was dissolved to obtain an undercoating agent.

### Example 4

The bulk powder of the compound A (3.5 g), D-mannitol (970.2 g), crosscarmelose sodium (52.5 g) and light anhydrous silicic acid (9.8 g) were put in a fluidized-bed granulation dryer (manufactured by Powrex Corp.), previously heated and mixed. The mixture was sprayed with a solution (816.7 g) of hydroxypropyl cellulose (49 g) to obtain a granule. The granule (930 g) was sized with a power mill (manufactured by Showa Kagaku Kikaikosakusho Co.) to obtain a sized granule. The obtained sized granule (899 g), crosscarmelose sodium (48.43 g) and magnesium stearate (9.57 g) were mixed with a tumbler mixer (manufactured by Showa Kagaku Kikaikosakusho Co.) to obtain mixed powder. The mixed powder (924 g) was compressed into a tablet with a tableting machine (manufactured by Kikusui Seisakusho Ltd.) to obtain a plain tablet.

### Example 5

The plain tablet obtained in Example 4 was sprayed with the undercoating agent obtained in Reference Example 3 so as to attain a coating of 15 mg/one tablet, in a film coating machine (manufactured by Freund). The undercoated tablet was then sprayed with the coating agent obtained in Reference Example 2 so as to attain a coating of 15 mg/one tablet to obtain a film-coated tablet.

### Example 6

The bulk powder of the compound A (3.5 g), D-mannitol (935.2 g), crosscarmelose sodium (52.5 g), light anhydrous silicic acid (9.8 g) and sodium ascorbate (35 g) were put in a fluidized-bed granulation dryer (manufactured by Powrex Corp.), previously heated and mixed. The mixture was sprayed with a solution (816.7 g) of hydroxypropyl cellulose (49 g) to obtain a granule. The granule (930 g) was sized with a power mill (manufactured by Showa Kagaku Kikaikosakusho Co.) to obtain a sized granule. The obtained sized granule (899 g), crosscarmelose sodium (48.43 g) and magnesium stearate (9.57 g) were mixed with a tumbler mixer (manufactured by Showa Kagaku Kikaikosakusho Co.) to obtain mixed powder. The mixed powder (924 g) was compressed into a tablet with a tableting machine (manufactured by Kikusui Seisakusho Ltd.) to obtain a plain tablet.

### Example 7

The plain tablet obtained in Example 6 was sprayed with the undercoating agent obtained in Reference Example 3 so as to attain a coating of 15 mg/one tablet, in a film coating machine (manufactured by Freund). The undercoated tablet was then sprayed with the coating agent obtained in Reference Example 2 so as to attain a coating of 15 mg/one tablet to obtain a film-coated tablet.

### Example 8

The bulk powder of the compound A (350 g), D-mannitol (588.7 g), crosscarmelose sodium (52.5 g), light anhydrous silicic acid (9.8 g) and sodium ascorbate (35 g) were put in a fluidized-bed granulation dryer (manufactured by Powrex Corp.), previously heated and mixed. The mixture was sprayed with a solution (816.7 g) of hydroxypropyl cellulose (49 g) to obtain a granule. The granule (930 g) was sized with a power mill (manufactured by Showa Kagaku Kikaikosakusho Co.) to obtain a sized granule. The obtained sized granule (899 g), crosscarmelose sodium (48.43 g) and magnesium stearate (9.57 g) were mixed with a tumbler mixer (manufactured by Showa Kagaku Kikaikosakusho Co.) to obtain mixed powder. The mixed powder (924 g) was compressed into a tablet with a tableting machine (manufactured by Kikusui Seisakusho Ltd.) to obtain a plain tablet.

### Example 9

The plain tablet obtained in Example 8 was sprayed with the undercoating agent obtained in Reference Example 3 so as to attain a coating of 15 mg/one tablet, in a film coating machine (manufactured by Freund). The undercoated tablet was then sprayed with the coating agent obtained in Reference Example 2 so as to attain a coating at 15 mg/one tablet to obtain a film-coated tablet.

### Example 10

The bulk powder of the compound A (350 g), D-mannitol (623.7 g), crosscarmelose sodium (52.5 g) and light anhydrous silicic acid (9.8 g) were put in a fluidized-bed granulation dryer (manufactured by Powrex Corp.), previously heated and mixed. The mixture was sprayed with a solution (816.7 g) of hydroxypropyl cellulose (49 g) to obtain a granule. The granule (930 g) was sized with a power mill (manufactured by Showa Kagaku Kikaikosakusho Co.) to obtain a sized granule. The obtained sized granule (899 g), crosscarmelose sodium (48.43 g) and magnesium stearate (9.57 g) were mixed with a tumbler mixer (manufactured by Showa Kagaku Kikaikosakusho Co.) to obtain mixed powder. The mixed powder (924 g) was compressed into a tablet with a tableting machine (manufactured by Kikusui Seisakusho Ltd.) to obtain a plain tablet.

### Example 11

The plain tablet obtained in Example 10 was sprayed with the undercoating agent obtained in Reference Example 3 so as to attain a coating of 15 mg/one tablet, in a film coating machine (manufactured by Freund). The undercoated tablet was then sprayed with the coating agent obtained in Reference Example 2 so as to attain a coating at 15 mg/one tablet to obtain a film-coated tablet.

### Reference Example 4

In purified water (1,440 g) titanium dioxide (48 g) and yellow ferric oxide (1.44 g) were dispersed. In purified water (2,880 g) hydroxypropylmethyl cellulose 2910 (TC-5) (358.56 g) and Macrogol 6000 (72 g) were dissolved. The resulting dispersion and the resulting solution were mixed to obtain a coating agent.

### Reference Example 5

In purified water (5,400 g) hydroxypropylmethyl cellulose 2910 (TC-5) (600 g) was dissolved to obtain an undercoating agent.

### Example 12

The bulk powder of the compound A (3.5 g), D-mannitol (847 g), crosscarmelose sodium (52.5 g) and sodium ascorbate (52.5 g) were put in a fluidized-bed granulation dryer (manufactured by Powrex Corp.), previously heated and mixed. The mixture was sprayed with a solution (525 g) of hydroxypropyl cellulose (31.5 g) to obtain a granule. The granule (846 g) was sized with a power mill (manufactured by Showa Kagaku Kikaikosakusho Co.) to obtain a sized granule. The obtained sized granule (817.8 g), cornstarch (121.8 g) and magnesium stearate (17.4 g) were mixed with a tumbler mixer (manufactured by Showa Kagaku Kikaikosakusho Co.) to obtain mixed powder. The mixed powder (924 g) was compressed into a tablet with a tableting machine (manufactured by Kikusui Seisakusho Ltd.) to obtain a plain tablet.

### Example 13

The plain tablet obtained in Example 12 was sprayed with the undercoating agent obtained in Reference Example 5 so as to attain a coating of 15 mg/one tablet, in a film coating machine (manufactured by Freund). The undercoated tablet was then sprayed with the coating agent obtained in Reference Example 4 so as to attain a coating at 12 mg/one tablet to obtain a film-coated tablet.

### Example 14

The plain tablet obtained in Example 12 was sprayed with the coating agent obtained in Reference Example 4 so as to attain a coating of 12 mg/one tablet, in a film coating machine (manufactured by Freund) to obtain a film-coated tablet.

### Experimental Example 2

The tablet obtained in Example 1 was put in a capped glass bottle and sealed. After storage at 60°C for 2 weeks, the contents of the compound A and the related substances in the tablet were measured. Similarly, the bulk powder used for production of the tablet in Example 1 was stored under the same conditions and then the contents of the compound A and the related substances were measured. A comparison of stabilities between the formulated tablet and the bulk powder was made.

Measurement of the contents of the compound A and the related substances was carried out by a HPLC method under the following conditions:
solvent: water/acetonitrile mixed solution (4 : 6),
measurement wavelength: 287 nm,
column: XTerra MS C18 3.5 µm 4.6 mm × 150 mm (manufactured by Waters Co., Ltd.),
mobile phase: a gradient of 10 mM ammonium acetate solution/acetonitrile mixed solution (4 : 3) and
acetonitril/10 mM ammonium acetate solution mixed solution (9 : 1), and
oven temperature: around 40°C.

As a result, as shown in Table 1, there was no significant change in the content of the compound A caused by formulation, but an increase of the related substances was remarkably suppressed by formulation, which confirms improvement in the stability.

**Table 1**

| Sample | Storage condition | Residual ratio (%) | Related substances (%) | | | | | |
|---|---|---|---|---|---|---|---|---|
| | | | A | B | C | D | E | F |
| bulk | initial | 100.0 | 0.46 | - | 0.24 | 0.32 | 0.13 | 3.01 |
| | 60°C 2W | 87.9 | 1.74 | 0.59 | 1.13 | 2.12 | 0.78 | 10.41 |
| Example | initial | 100.0 | 0.25 | - | 0.25 | 0.40 | 0.12 | 3.62 |
| 1 | 60°C 2W | 86.8 | 1.05 | 0.52 | 1.63 | 1.90 | 0.15 | 7.93 |
| Initial: Immediately after production, 60°C | | | | | | | | |
| 2W: After 2-week storage at 60°C | | | | | | | | |

### Experimental Example 3

The 1 mg tablet that did not contain sodium ascorbate obtained in Example 5 and the 1 mg tablet containing sodium ascorbate obtained in Example 7 were dried in vacuum and placed within desiccators containing a saturated potassium carbonate solution for 3 days to control the humidity. The equilibrium moisture content (ERH) of each tablet was measured by the following method. The results were 3.0% and 3.4%, respectively.

### (Method for measuring equilibrium moisture content of formulated agent)

The measurement was carried out at 20 to 25°C by using 5 to 30 plain tablets or film-coated tablets and Rotronic Hygroskop DT (manufactured by Rotronic Co.).

These tablets were put in a capped glass bottle and sealed. After storage at 40°C for 1 month, the contents of the compound A and the related substances were measured. The content of the compound A was measured in the same manner as the measurement method in Experimental Example 2, except that a water/acetonitrile mixed solution (4 : 6) was used as a solvent. The measurement of the related substances was carried out in the same manner as Experimental Example 3.

Since the related substance A could not be separated from sodium ascorbate in the measurement, it was not evaluated. As a result, as shown in Table 2, the tablet containing sodium ascorbate did not have a decrease in the content of the compound A and an increase of the related substances. In addition, it was confirmed that sodium ascorbate incorporated in a tablet suppressed decomposition of the main drug during production of the tablet, which led to stabilization of the tablet, based on a comparison of the initial contents of the related substances between the tablets of Example 5 and Example 7, wherein both tablets were produced from the same bulk.

**Table 2**

| Sample | Storage condition | Residual ratio (%) | Related substances (%) | | | | |
|---|---|---|---|---|---|---|---|
| | | | B | C | D | E | F |
| Example | initial | 100.0 | - | 1.40 | 1.74 | - | 9.29 |
| 5 | 40°C 1M | 76.8 | 1.29 | 7.08 | 6.53 | - | 15.02 |
| Example | initial | 100.0 | - | 0.36 | 0.57 | - | 4.69 |
| 7 | 40°C 1M | 89.3 | - | 2.20 | 2.00 | - | 10.23 |
| Initial: Immediately after production, | | | | | | | |
| 40°C 1M: After 1-month storage at 40°C | | | | | | | |

The contents of the related substances A to F in the bulk used were as follows:
related substance A: 0.31%, related substance B: 0.08%,
related substance C: 0.40%, related substance D: 0.43%,
related substance E: 0.08%, related substance A: 2.69%.

### Experimental Example 4

The 1 mg tablet obtained in Example 5 was dried in vacuum. A portion of the tablets was placed within a desiccator containing a saturated potassium carbonate solution for 3 days to control the humidity. The equilibrium moisture content (ERH) of each sample was measured by the above-mentioned method. The results were 3.0% for the vacuum-dried sample and 46.6% for the humidity-controlled sample. Each sample was put in a capped glass bottle and sealed. After storage at 40°C for 1 month, the contents of the compound A and the related substances were measured in the same manner as Experimental Example 3. As a result, as shown in Table 3, the sample with more than ERH 10% had less decrease in the content of the compound A and less increase of the related substances, as compared with the sample with less than ERH 10%.

**Table 3**

| Sample | Storage condition | Residual ratio (%) | Related substance (%) | | | | | |
|---|---|---|---|---|---|---|---|---|
| | | | A | B | C | D | E | F |
| Vacuum- | initial | 100.0 | 0.70 | - | 1.40 | 1.74 | - | 9.29 |
| dried product (ERH: 3.0%) | 40°C 1M | 76.8 | 5.12 | 1.29 | 7.08 | 6.53 | - | 15.02 |
| Humidity- | initial | 100.0 | 0.70 | - | 1.40 | 1.74 | - | 9.29 |
| controlled product (ERH: 46.6%) | 40°C 1M | 83.5 | 2.55 | 0.88 | 5.62 | 5.26 | - | 12.94 |
| Initial: Immediately after production, | | | | | | | | |
| 40°C 1M: After 1-month storage at 40°C | | | | | | | | |

### Experimental Example 5

The film-coated tablet obtained in Example 13, which was coated with an anchor coating and then with a usual film coating, and the film-coated tablet obtained in Example 14, which was coated with a usual film coating, were put in glass bottles and sealed. After storage at 40°C/75% RH for 3 months, the contents of the compound A and the related substances were measured in the same manner as Experimental Example 3. Since the related substance A could not be separated from sodium ascorbate in the measurement, it was not evaluated. As a result, as shown in Table 4, a decrease in the content of the compound A and an increase of the related substances were lessened by applying the anchor coating.

**Table 4**

| Sample | Storage condition | Residual ratio (%) | Related substances (%) | | | | |
|---|---|---|---|---|---|---|---|
| | | | B | C | D | E | F |
| Example 13 | initial | 100.0 | - | 0.39 | 0.27 | - | 3.07 |
| (with anchor | 40°C/75% | 94.2 | - | - | 1.06 | - | 6.82 |
| coating) | RH3M | | | | | | |
| Example 14 | initial | 100.0 | - | 0.44 | 0.55 | - | 3.23 |
| (without | 40°C/75% | 92.3 | 0.05 | - | 1.49 | 0.17 | 7.70 |
| anchor | RH3M | | | | | | |
| coating) | | | | | | | |
| Initial: Immediately after production, | | | | | | | |
| 40°C/75% RH 3M: After 3-month storage at 40°C and 75% RH | | | | | | | |

### Experimental Example 6

The tablet obtained in Example 5 was dried in vacuum. The equilibrium moisture content (ERH) of the tablet was measured by the above-mentioned method. The result was 3.0%. The vacuum-dried sample was put in two glass bottles. One bottle was sealed with a cap as it was. The other was sealed with a cap after the inside was replaced with nitrogen gas. After the both bottles were kept at 40°C for 1 month, the contents of the compound A and the related substances were measured in the same manner as Experimental Example 3. As a result, as shown in Table 5, no significant decrease in the content of the compound A and no increase of the related substances were observed for the sample kept under nitrogen-replaced condition.

**Table 5**

| Sample | Storage condition | Residual ratio (%) | Related substances (%) | | | | | |
|---|---|---|---|---|---|---|---|---|
| | | | A | B | C | D | E | F |
| without | initial | 100.0 | 0.70 | - | 1.40 | 1.74 | - | 9.29 |
| replacement | 40°C 1M | 76.8 | 5.12 | 1.29 | 7.08 | 6.53 | - | 15.02 |
| with | | | | | | | | |
| nitrogen | | | | | | | | |
| with | initial | 100.0 | 0.70 | - | 1.40 | 1.74 | - | 9.29 |
| replacement | 40°C 1M | 97.6 | 2.07 | - | 1.68 | 1.87 | - | 10.58 |
| with | | | | | | | | |
| nitrogen | | | | | | | | |
| Initial: Immediately after production, | | | | | | | | |
| 40°C 1M: After 1-month storage at 40°C | | | | | | | | |

### Experimental Example 7

The tablet obtained in Example 5 was dried in vacuum. The equilibrium moisture content (ERH) of the tablet was measured by the above-mentioned method. The result was 3.0%. The vacuum-dried sample was put in two glass bottles. One bottle was sealed with a cap as it was. The other bottle was sealed with a cap after an oxygen scavenger (Ageless (Z-20PT): manufactured by Mitsubishi Gas Chem. Co., Ltd.) was put in the bottle. After the both bottles were kept at 40°C for 1 month, the contents of the compound A and the related substances were measured in the same manner as Experimental Example 3. As a result, as shown in Table 6, no decrease in the content of the compound A and no remarkable increase of the related substances were observed for the sample kept together with the oxygen scavenger, Ageless Z-20 PT.

**Table 6**

| Sample | Storage condition | Residual ratio (%) | Related substances (%) | | | | | |
|---|---|---|---|---|---|---|---|---|
| | | | A | B | C | D | E | F |
| without | initial | 100.0 | 0.70 | - | 1.40 | 1.74 | - | 9.29 |
| oxygen | 40°C 1M | 76.8 | 5.12 | 1.29 | 7.08 | 6.53 | - | 15.02 |
| scavenger | | | | | | | | |
| with | initial | 100.0 | 0.70 | - | 1.40 | 1.74 | - | 9.29 |
| oxygen | 40°C 1M | 99.8 | 0.86 | - | 1.57 | 1.79 | - | 7.15 |
| scavenger | | | | | | | | |
| Initial: Immediately after production, | | | | | | | | |
| 40°C 1M: After 1-month storage at 40°C | | | | | | | | |

### Experimental Example 8

The plain tablet produced in Example 12, the film-coated tablet produced in Example 13, which was coated with an anchor coating and then with a usual film coating, and these tablets covered with aluminum foil for shielding from light were exposed to the light of a xenon lamp at 100,000 lux for 12 hours (1,200,000 lux·h) by using a light resistance tester (manufactured by Suga Test Instruments Co., Ltd.) and then the contents of the compound A and the related substances were measured. The contents of the compound A and the related substances were measured in the same manner as Experimental Example 2. Since the related substance A could not be separated from sodium ascorbate in the measurement, it was not evaluated. As a result, as shown in Table 7, a decrease in the content of the compound A and an increase of the related substances were suppressed by applying the film-coating.

**Table 7**

| Sample | Storage condition | Content (%) | Related substances (%) | | | | |
|---|---|---|---|---|---|---|---|
| | | | B | C | D | E | F |
| Example 13 | exposure to | 97.8 | - | 0.69 | 0.81 | - | 3.39 |
| | light | | | | | | |
| | shielding | 97.9 | - | 0.47 | 0.59 | - | 2.94 |
| | from light | | | | | | |
| Example 12 | exposure to | 90.4 | - | 1.25 | 1.43 | - | 3.91 |
| | light | | | | | | |
| | shielding | 98.1 | - | 0.44 | 0.54 | - | 2.84 |
| | from light | | | | | | |

### Experimental Example 9

The 1 mg tablet containing sodium ascorbate obtained in Example 7 and the 100 mg tablet containing sodium ascorbate obtained in Example 9 were dried in vacuum and then placed within desiccators containing a saturated potassium carbonate solution for 3 days to control the humidity. The equilibrium moisture contents (ERH) of each sample was measured by the above-mentioned method. The results were 46.2% and 44.5%, respectively. These samples were put in glass bottles. The bottles were sealed with caps after oxygen scavengers (Ageless (Z-20PT): manufactured by Mitsubishi Gas Chem. Co., Ltd.) were put therein. After the bottles were kept at 40°C for 1 month, the content of the compound A and the related substances were measured in the same manner as Experimental Example 3. The equilibrium moisture contents (ERH) were 65.3% and 65.1%, respectively, after the storage. As a result, as shown in Table 8, there were no significant decrease in the content of the compound A and no significant increase of the related substances, which show that these tablets were stable. In addition, each sample was put in a glass bottle, which was sealed with a cap and kept at room temperature (23 to 28°C). During the storage for 21 days, each bottle was opened to take a tablet from the bottle and then sealed with a cap again every day. The content of the compound A and the related substances in the tablet taken from the bottle on the 21st day were measured. The equilibrium moisture contents (ERH) were 42.0% and 38.3%, respectively. As a result, as shown in Table 9, there were no significant decrease in the content of the compound A and no significant increase of the related substances, which confirmed that these tablets were stable even after opening the bottles. These evaluations were carried out for the related substances C, D, and F which showed considerable changes.

**Table 8**

| Sample | Storage condition | Residual ratio (%) | Related substances (%) | | |
|---|---|---|---|---|---|
| | | | C | D | F |
| Example 7 | initial | 100.0 | 0.36 | 0.57 | 4.69 |
| | 40°C 1M | 101.1 | 0.59 | 0.64 | 3.58 |
| Example 9 | initial | 100.0 | 0.39 | 0.23 | 3.51 |
| | 40°C 1M | 102.4 | 0.24 | - | 3.65 |
| Initial: Immediately after production, | | | | | |
| 40°C 1M: After 1-month storage at 40°C | | | | | |

**Table 9**

| Sample | Storage condition | Residual ratio (%) | Related substances (%) | | |
|---|---|---|---|---|---|
| | | | C | D | F |
| Example 7 | initial | 100.0 | 0.36 | 0.57 | 4.69 |
| | after 21 | 99.8 | 0.56 | 0.63 | 3.24 |
| | days | | | | |
| Example 9 | initial | 100.0 | 0.39 | 0.23 | 3.51 |
| | after 21 | 97.8 | 0.59 | 0.67 | 3.73 |
| | days | | | | |
| Initial: Immediately after production | | | | | |

### Experimental Example 10

The 1 mg tablet containing sodium ascorbate obtained in Example 7 and the 100 mg tablet containing sodium ascorbate obtained in Example 9 were dried in vacuum and then placed within desiccators containing a saturated potassium carbonate solution for 3 days to control the humidity. The equilibrium moisture content (ERH) of each sample was measured by the above-mentioned method. The results were 46.2% and 44.5%, respectively. These samples were put in glass bottles. The bottles were sealed with caps after the insides were replaced with nitrogen gas. After the bottles were kept at 40°C for 1 month, the contents of the compound A and the related substances were measured in the same manner Experimental Example 3. As a result, as shown in Table 10, there were no significant decrease in the content of the compound A and no significant increase of the related substances after the storage, which confirmed that these tablets were stable. These evaluations were carried out for the related substances C, D, and F which showed considerable changes.

**Table 10**

| Sample | Storage condition | Residual ratio (%) | Related substances (%) | | |
|---|---|---|---|---|---|
| | | | C | D | F |
| Example 7 | initial | 100.0 | 0.36 | 0.57 | 4.69 |
| | 40°C 1M | 98.3 | 0.57 | 0.63 | 3.30 |
| Example 9 | initial | 100.0 | 0.39 | 0.23 | 3.51 |
| | 40°C 1M | 106.3 | 0.39 | 0.25 | 4.02 |
| Initial: Immediately after production, | | | | | |
| 40°C 1M: After 1-month storage at 40°C | | | | | |

### Experimental Example 11

The 1 mg tablet containing no sodium ascorbate obtained in Example 5 and the 100 mg tablet that did not contain sodium ascorbate obtained in Example 11 were dried in vacuum and then placed within desiccators containing a saturated potassium carbonate solution for 3 days to control the humidity. The equilibrium moisture content (ERH) of each sample was measured by the above-mentioned method. The results were 46.6% and 37.4%, respectively. These samples were put in glass bottles. The bottles were sealed with caps after oxygen scavengers (Ageless (Z-20PT): manufactured by Mitsubishi Gas Chem. Co., Ltd.) were put therein. After the bottles were kept at 40°C for 1 month, the content of the compound A and the related substances were measured in the same manner as Experimental Example 3. The equilibrium moisture contents (ERH) were 65.2% and 66.9%, respectively, after the storage. As a result, as shown in Table 11, there were no significant decrease in the content of the compound A and no significant increase of the related substances after the storage, which confirmed that these tablets were stable. These evaluations were carried out for the related substances C, D, and F which showed considerable changes.

**Table 11**

| Sample | Storage condition | Residual ratio (%) | Related substances (%) | | |
|---|---|---|---|---|---|
| | | | C | D | F |
| Example 5 | initial | 100.0 | 1.40 | 1.74 | 9.29 |
| | 40°C 1M | 99.8 | 1.73 | 1.86 | 6.77 |
| Example | initial | 100.0 | 2.30 | 0.39 | 4.43 |
| 11 | 40°C 1M | 103.0 | 0.30 | 0.40 | 3.97 |
| Initial: Immediately after production, | | | | | |
| 40°C 1M: After 1-month storage at 40°C | | | | | |

### Experimental Example 12

The 1 mg tablet containing no sodium ascorbate obtained in Example 5 and the 100 mg tablet that did not contain sodium ascorbate obtained in Example 11 were dried in vacuum and then placed within desiccators containing a saturated potassium carbonate solution for 3 days to control the humidity. The equilibrium moisture content (ERH) of each sample was measured by the above-mentioned method. The results were 46.6% and 37.4%, respectively. These samples were put in glass bottles. The bottles were sealed with caps after the insides were replaced with nitrogen gas. After the bottles were kept at 40°C for 1 month, the contents of the compound A and the related substances were measured in the same manner as Experimental Example 3. As a result, as shown in Table 12, there were no significant decrease in the content of the compound A and no significant increase of the related substances after the storage, which confirmed that these tablets were stable. These evaluations were carried out for the related substances C, D, and F which showed considerable changes.

**Table 12**

| Sample | Storage condition | Residual ratio (%) | Related substances (%) | | |
|---|---|---|---|---|---|
| | | | C | D | F |
| Example 5 | initial | 100.0 | 1.40 | 1.74 | 9.29 |
| | 40°C 1M | 96.3 | 2.23 | 2.27 | 9.72 |
| Example | initial | 100.0 | 2.30 | 0.39 | 4.43 |
| 11 | 40°C 1M | 105.1 | 0.31 | 0.52 | 4.18 |
| Initial: Immediately after production, | | | | | |
| 40°C 1M: After 1-month storage at 40°C | | | | | |

### Reference Example 6

In purified water (800 g) titanium dioxide (45.0 g), yellow ferric oxide (1.80 g) and red ferric oxide (1.80 g) were dispersed. In purified water (1,700 g) hydroxypropylmethyl cellulose 2910 (TC-5) (206.4 g) and Macrogol 6000 (45.0 g) were dissolved. The resulting dispersion, the resulting solution and purified water (200 g) were mixed to obtain a coating agent.

### Reference Example 7

In purified water (1,350 g) hydroxypropylmethyl cellulose 2910 (TC-5) (150 g) was dissolved to obtain an undercoating agent.

### Example 15

The bulk powder of the compound A (82.6 g), D-mannitol (4208.6 g), crosscarmelose sodium (240.0 g), light anhydrous silicic acid (44.8 g) and sodium ascorbate (160.0 g) were put in a fluidized-bed granulation dryer (manufactured by Powrex Corp.), previously heated and mixed. The mixture was sprayed with a solution (3733.3 g) of hydroxypropyl cellulose (224.0 g) to obtain a granule. The granule (4495 g) was sized with a power mill (manufactured by Showa Kagaku Kikaikosakusho Co.) to obtain a sized granule. The obtained sized granule (4185 g), crosscarmelose sodium (225.5 g) and magnesium stearate (44.6 g) were mixed with a tumbler mixer (manufactured by Showa Kagaku Kikaikosakusho Co.) to obtain mixed powder. The mixed powder (4207.5 g) was compressed into a tablet with a tableting machine (manufactured by Kikusui Seisakusho Ltd.) to obtain a plain tablet.

### Example 16

The plain tablet obtained in Example 15 was sprayed with the undercoating agent obtained in Reference Example 7 so as to attain a coating of 15 mg/one tablet, in a film coating machine (manufactured by Freund). The undercoated tablet was then sprayed with the coating agent obtained in Reference Example 6 so as to attain a coating of 15 mg/one tablet to obtain a film-coated tablet.

### Example 17

The plain tablet obtained in Example 15 was sprayed with the coating agent obtained in Reference Example 6 so as to attain a coating of 15 mg/one tablet, in a film coating machine (manufactured by Freund) to obtain a film-coated tablet.

### Experimental Example 13

The film-coated tablet obtained in Example 16, which was coated with an anchor coating and then with a usual film coating, and the film-coated tablet obtained in Example 17, which was coated with a usual film coating, were put in glass bottles. The bottles were sealed with caps, after small glass bottles containing a saturated sodium bromide solution were placed therein and then the insides were replaced with nitrogen gas. After the bottles were kept at 40°C for 2 months, the contents of the compound A and the related substances were measured by a HPLC method.

The measurement of the compound A and the related substances B to F was carried out under the following conditions:
column: XTerra MS C18 3.5 µm 4.6 mm × 150 mm (manufactured by Waters Co., Ltd.),
mobile phase: a gradient of 10 mM ammonium acetate solution/acetonitrile mixed solution (4 : 3) and acetonitril/10 mM ammonium acetate solution mixture (9 : 1).

The measurement of the related substances G and H was carried out under the following conditions:
column: CAPCELL PAK C18 MG 5 µm 4.6 mm × 150 mm (manufactured by Shiseido Co., Ltd),
mobile phase: a gradient of 10 mM ammonium acetate solution/acetonitrile mixed solution (50 : 1) and acetonitril/10 mM ammonium acetate solution mixture (9 : 1).

Both cases were carried out under the following conditions:
measurement wavelength: 287 nm,
oven temperature: around 25°C,
solvent: acetonitril/10 mM ammonium acetate solution mixture (7 : 3).

With respect to the related substance A, it was found that the substance could be separated into the related substances G and related substances H by a new testing method.

The equilibrium moisture contents (ERH) of the samples were measured by the following method. The results were 23.3% at the initial and 53.9% after the storage for the tablet of Example 16 and 25.0% at the initial and 51.7% after the storage for the tablet of Example 17.

### (Method for measuring equilibrium moisture content of formulated agent)

The measurement was carried out at 20 to 25°C by using 5 to 30 plain tablets or film-coated tablets and Rotronic Hygroskop DT (manufactured by Rotronic Co.).

As a result, as shown in Table 13, there were no significant difference in the residual ratios and no significant increase or decrease in the related substances depending on the existence of the anchor coating.

**Table 13**

| Sample | Storage condition | Residual ratio (%) | Related substances (%) | | | | | | |
|---|---|---|---|---|---|---|---|---|---|
| | | | B | C | D | E | F | G | H |
| Example | initial | 100.0 | 0.12 | 0.59 | 0.60 | 0.09 | 5.50 | 0.25 | 0.15 |
| 16 | 40°C/ | 99.2 | 0.14 | 0.66 | 0.66 | - | 5.43 | 0.16 | - |
| (with | nitrogen | | | | | | | | |
| anchor | - | | | | | | | | |
| coating | replaced | | | | | | | | |
| ) | 2M | | | | | | | | |
| Example | initial | 100.0 | 0.12 | 0.56 | 0.57 | 0.10 | 5.43 | 0.24 | 0.14 |
| 17 | 40°C/ | 99.4 | 0.13 | 0.65 | 0.64 | - | 5.36 | 0.14 | - |
| (without | nitrogen | | | | | | | | |
| | - | | | | | | | | |
| anchor | replaced | | | | | | | | |
| coating) | 2M | | | | | | | | |
| Initial: Immediately after production, | | | | | | | | | |
| 40°C/nitrogen-replaced 2M: After 2-month storage at 40°C in a sealed container wherein the saturated potassium acetate solution was enclosed and of which the inside was replaced with nitrogen gas. | | | | | | | | | |

### Example 18

The bulk powder of the compound A (1315 g), D-mannitol (2297 g), crosscarmelose sodium (195 g) and sodium ascorbate (130 g) were put in a fluidized-bed granulation dryer (manufactured by Powrex Corp.), previously heated and mixed. The mixture was sprayed with a solution (2600 g) of hydroxypropyl cellulose (130 g) to obtain a granule. The granule was sized with a power mill (manufactured by Showa Kagaku Kikaikosakusho Co.) to obtain a sized granule. The obtained sized granule (930 g), crosscarmelose sodium (50.1 g) and magnesium stearate (9.9 g) were mixed to obtain mixed powder. The mixed powder was compressed into a tablet with a tableting machine (manufactured by Kikusui Seisakusho Ltd.) to obtain a plain tablet.

### Example 19

The plain tablet obtained in Example 18 was sprayed with the coating agent obtained in Reference Example 6 so as to attain a coating of 15 mg/one tablet, in a film coating machine (manufactured by Freund) to obtain a film-coated tablet.

### Experimental Example 14

The tablet obtained in Example 19 was put in a glass bottle. The bottle was sealed with caps, after a small glass bottle containing a saturated potassium acetate solution was placed therein and then the inside was replaced with nitrogen gas. After the bottle was kept at 40°C for 2 months, the contents of the compound A and the related substances were measured in the same manner as Experimental Example 12. With respect to the related substance A, it was found that the substance could be separated into the related substances G and related substances H by a new testing method.

The equilibrium moisture contents (ERH) of the sample was measured by the following method. The results were 17.3% at the initial and 19.85% after the storage.

### (Method for measuring equilibrium moisture content of formulated agent)

The measurement was carried out at 20 to 25°C by using 5 to 30 plain tablets or film-coated tablets and Rotronic Hygroskop DT (manufactured by Rotronic Co.).

As a result, as shown in Table 14, neither a decrease in the content of the compound A nor an increase of the related substances was observed.

**Table 14**

| Sample | Storage condition | Residual ratio (%) | Related substances (%) | | | | | | |
|---|---|---|---|---|---|---|---|---|---|
| | | | B | C | D | E | F | G | H |
| Example | initial | 100.0 | - | 0.13 | 0.14 | - | 2.18 | - | - |
| 19 | 40°C/ | 101.5 | - | 0.12 | 0.13 | - | 2.23 | - | - |
| | nitrogen- | | | | | | | | |
| | replaced | | | | | | | | |
| | 2M | | | | | | | | |
| Initial: Immediately after production, 40°C/nitrogen-replaced2M: After 2-month storage at 40°C in a sealed container wherein the saturated potassium acetate solution was enclosed and of which the inside was replaced with nitrogen gas. | | | | | | | | | |

### Reference Example 8

In purified water (1600 g) titanium dioxide (90.0 g), yellow ferric oxide (3.60 g) and red ferric oxide (3.60 g) were dispersed. In purified water (3400 g) hydroxypropylmethyl cellulose 2910 (TC-5) (412.8 g) and Macrogol 6000 (90.0 g) were dissolved. The resulting dispersion, the resulting solution and purified water (400 g) were mixed to obtain a coating agent.

### Example 20

The bulk powder of the compound A (407 g), D-mannitol (3994 g), crosscarmelose sodium (240 g) and sodium ascorbate (160 g) were put in a fluidized-bed granulation dryer (manufactured by Powrex Corp.), previously heated and mixed. The mixture was sprayed with a solution (3200 g) of hydroxypropyl cellulose (160 g) to obtain a granule. The granule (4588 g) was sized with a power mill (manufactured by Showa Kagaku Kikaikosakusho Co.) to obtain a sized granule. This batch process was carried out 2 times. The obtained sized granule (8742 g), crosscarmelose sodium (471 g) and magnesium stearate (93.1 g) were mixed with a tumbler mixer (manufactured by Showa Kagaku Kikaikosakusho Co.) to obtain mixed powder. The mixed powder (8976 g) was compressed into a tablet with a tableting machine (manufactured by Kikusui Seisakusho Ltd.) to obtain a plain tablet.

### Example 21

The plain tablet obtained in Example 20 was sprayed with the coating agent obtained in Reference Example 8 so as to attain a coating of 15 mg/one tablet, in a film coating machine (manufactured by Freund) to obtain a film-coated tablet.

### Example 22

The bulk powder of the compound A (1626 g), D-mannitol (2774 g), crosscarmelose sodium (240 g) and sodium ascorbate (160 g) were put in a fluidized-bed granulation dryer (manufactured by Powrex Corp.), previously heated and mixed. The mixture was sprayed with a solution (3200 g) of hydroxypropyl cellulose (160 g) to obtain a granule. The granule (4588 g) was sized with a power mill (manufactured by Showa Kagaku Kikaikosakusho Co.) to obtain a sized granule. This batch process was carried out 2 times. The obtained sized granule (8742 g), crosscarmelose sodium (471 g) and magnesium stearate (93.1 g) were mixed with a tumbler mixer (manufactured by Showa Kagaku Kikaikosakusho Co.) to obtain mixed powder. The mixed powder (8976 g) was compressed into a tablet with a tableting machine (manufactured by Kikusui Seisakusho Ltd.) to obtain a plain tablet.

### Example 23

The plain tablet obtained in Example 22 was sprayed with the coating agent obtained in Reference Example 8 so as to attain a coating of 15 mg/one tablet, in a film coating machine (manufactured by Freund) to obtain a film-coated tablet.

### Industrial Applicability

According to the present invention, a stable pharmaceutical composition wherein a fused nitrogen-containing heterocyclic compound unstable to oxygen, particularly the above-mentioned compound (I) or (II) is stabilized can be obtained.

## Claims

1. A solid composition containing a fused nitrogen-containing heterocyclic compound unstable to oxygen, which is stabilized by
[1] maintaining an equilibrium moisture content of 10% or above in the solid composition and/or
[2] incorporating ascorbic acid or a salt thereof in the solid composition.

2. The solid composition according to claim 1, wherein the fused nitrogen-containing heterocyclic compound is a compound represented by the formula: 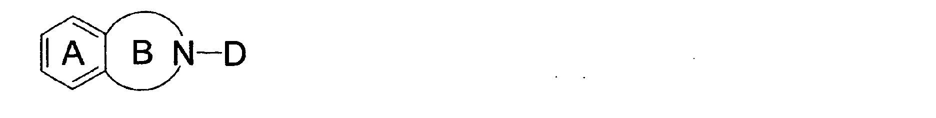 wherein, ring A is an optionally substituted benzene ring; ring B is a 4 to 7-membered nitrogen-containing heterocyclic ring which may be optionally substituted with halogen, an optionally substituted heterocyclic ring or an optionally substituted hydrocarbon group in addition to D; and D is a hydrogen atom, a heterocyclic group which may be optionally substituted and may optionally have a fused ring, or an optionally substituted hydrocarbon group, or a salt thereof.

3. The solid composition according to claim 1, wherein the fused nitrogen-containing heterocyclic compound is an isoindoline compound.

4. The solid composition according to claim 3, wherein the fused nitrogen-containing heterocyclic compound is (R)-(+)-5,6-dimethoxy-2-[2,2,4,6,7-pentamethyl-3-(4-methylphenyl)-2,3-dihydro-1-benzofuran-5-yl]isoindoline, (R)-(+)-5,6-dimethoxy-2-[2,2,4,6,7-pentamethyl-3-(1-methylethylphenyl)-2,3-dihydro-1-benzofuran-5-yl]isoindoline, (R)-(+)-5,6-dimethoxy-2-[2,2,4,6,7-pentamethyl-3-(4-bromophenyl)-2,3-dihydro-1-benzofuran-5-yl]isoindoline, or a salt thereof.

5. The solid composition according to claim 2, which is coated with a film for protection from light.

6. The solid composition according to claim 5, which is precoated with a film that does not contain a light blocking agent.

7. A packed product obtained by packing the solid composition according to any one of claims 1 to 6 in one or more packaging forms selected from oxygen permeation-suppressing package, gas-replacement package, vacuum package and sealing package with an oxygen scavenger.

8. A packed product obtained by packing a solid composition in nitrogen-replacement package, wherein the solid composition comprises a compound represented by the formula: 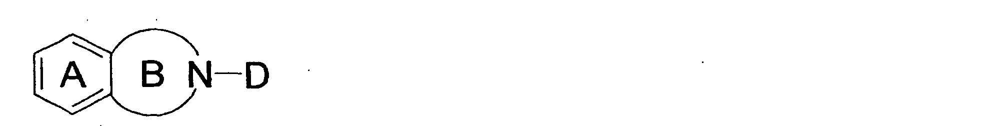 wherein, ring A is an optionally substituted benzene ring; ring B is a 4 to 7-membered nitrogen-containing heterocyclic ring which may be optionally substituted with halogen, an optionally substituted heterocyclic ring or an optionally substituted hydrocarbon group in addition to D; and D is a hydrogen atom, a heterocyclic group which may be optionally substituted and may optionally have a fused ring, or an optionally substituted hydrocarbon group, or a salt thereof, and ascorbic acid or a salt thereof; is coated with a film for protection from light without being precoated with a film; and has an equilibrium moisture content of 10% or above.

9. A method for stabilizing a solid composition containing a fused nitrogen-containing heterocyclic compound unstable to oxygen, which comprises
[1] maintaining an equilibrium moisture content of 10% or above in the solid composition,
[2] incorporating ascorbic acid or a salt thereof in the solid composition, and/or
[3] packing the solid composition in one or more packaging forms selected from oxygen permeation-suppressing package, gas-replacement package, vacuum package and sealing package with an oxygen scavenger.

10. The method according to claim 9, wherein the fused nitrogen-containing heterocyclic compound is a compound represented by the formula: 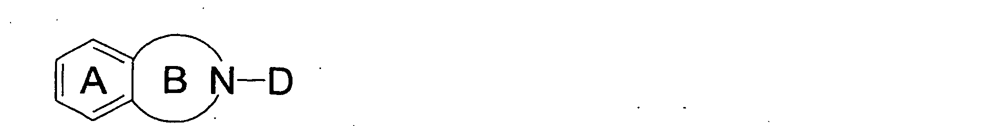 wherein, ring A is an optionally substituted benzene ring; ring B is a 4 to 7-membered nitrogen-containing heterocyclic ring which may be optionally substituted with halogen, an optionally substituted heterocyclic ring or an optionally substituted hydrocarbon group in addition to D; and D is a hydrogen atom, a heterocyclic group which may be optionally substituted and may optionally have a fused ring, or an optionally substituted hydrocarbon group, or a salt thereof.

11. The method according to claim 9, wherein the fused nitrogen-containing heterocyclic compound is an isoindoline compound.

12. The stabilization method according to claim 9, wherein the fused nitrogen-containing heterocyclic compound is (R)-(+)-5,6-dimethoxy-2-[2, 2,4,6,7-pentamethyl-3-(4-methylphenyl)-2,3-dihydro-1-benzofuran-5-yl]isoindoline, (R)-(+)-5,6-dimethoxy-2-[2,2,4,6, 7-pentamethyl-3-(1-methylethylphenyl)-2,3-dihydro-1-benzofuran-5-yl] isoindoline, (R)-(+)-5,6-dimethoxy-2-[2,2,4,6,7-pentamethyl-3-(4-bromophenyl)-2,3-dihydro-1-benzofuran-5-yl]isoindoline or a salt thereof.

13. A stabilized solid composition which comprises [1] a compound unstable to oxygen and [2] an antioxidant that is less oxidizable than said compound and wherein an equilibrium moisture content of 10% or above is maintained.

14. A packed product obtained by packing the composition according to claim 13 in one or more packaging forms selected from oxygen permeation-suppressing package, gas-replacement package, vacuum package, and sealing package with an oxygen scavenger.

15. A method for stabilizing a solid composition containing a compound unstable to oxygen, which comprises
[1] maintaining an equilibrium moisture content of 10% or above in the solid composition,
[2] incorporating an antioxidant that is less oxidizable than the compound in the solid composition, and/or
[3] packing the solid composition in one or more packaging forms selected from oxygen permeation-suppressing package, gas-replacement package, vacuum package, and oxygen scavenger-enclosed package.
